# EUROPEAN PATENT APPLICATION

(11) **EP 2 253 642 A1**
(43) Date of publication of application: **24.11.2010**
(21) Application number: 10171714.8
(22) Date of filing: 25.12.1998
(51) Int. Cl.: C07K 14/47, C12N 15/12, C07K 16/18, C12P 21/08, A61K 38/17

(54) **Polypeptides, cDNAs encoding the same and utilization thereof**

(30) Priority: 26.12.1997 JP 35881197
(62) Divisional of application: 05109270.8
(71) Applicant: ONO Pharmaceutical Co., Ltd., Osaka-shi, Osaka 541-8526 (JP)
(72) Inventor: Shibayama, Shiro, Osaka Osaka 618-8585 (JP); Tada, Hideaki, Osaka Osaka 618-8585 (JP); Fukushima, Daikichi, Osaka Osaka 618-8585 (JP)
(74) Representative: Schön, Christoph

(57) **Abstract**

A new polypeptide prepared from produced from a human adult brain tissue, a cell line derived from a human brain tissue, a cell line derived from human bone marrow, and an endothelial cell line of vein derived from human umbilical cord and a process for preparation thereof; a cDNA encoding the polypeptide; a fragment selectively hybridizing with the sequence of the cDNA; a replication or expression plasmid containing the cDNA integrated thereinto; a host cell transformed with plasmid; an antibody against the polypeptide; and a pharmaceutical composition containing the polypeptide or the antibody.

## Description

### Technical Field

The present invention relates to novel polypeptides, a process for preparation thereof, cDNAs encoding the polypeptide, vectors containing the cDNA, host cells transformed with the vector, antibodies against the polypeptide, and pharmaceutical compositions containing the polypeptide or the antibody.

### Technical Background

Until now, when one skilled in-the art intends to obtain a particular polypeptide or a cDNA encoding it, he/she generally utilizes methods by confirming an aimed biological activity in a tissue or in a cell medium, isolating and purifying the polypeptide and then cloning a gene or methods by "expression-cloning" with the guidance of the biological activity. However, physiologically active polypeptides in living body have often many kinds of activities. Therefore, it happens increasingly that after cloning a gene, the isolated gene is found to be identical to that encoding a polypeptide already known. In addition, some factors could be generated in only a very slight amount and/or under specific conditions and it makes difficult to isolate and to purify the factor and to confirm its biological activity.

Recent rapid developments in techniques for constructing cDNAs and sequencing techniques have made it possible to quickly sequence a large amount of cDNAs. By utilizing these techniques, a process, which comprises constructing cDNA library using various cells or tissues, cloning the cDNA at random, identifying the nucleotide sequences thereof, and expressing novel polypeptides encoded thereby, is now in progress. Although this process is advantageous in that a gene can be cloned and information regarding its nucleotide sequence can be obtained without any biochemical or genetic analysis, the target gene can be discovered thereby only accidentally in many cases.

The present inventors have studied a cloning method to isolate genes encoding proliferation and/or differentiation factors functioning in hematopoietic systems and immune systems. Focusing their attention on the fact that most of the secretory proteins, such as proliferation and/or differentiation factors (for example, various cytokines *etc*.) and membrane proteins such as receptors thereof (hereafter these proteins will be referred to generally as secretory proteins and the like), have sequences called signal peptides in the N-termini, the inventors have conducted extensive studies on a process for efficiently and selectively cloning a gene encoding a signal peptide. Finally, we have successfully developed a method which can easily select a cDNA encoding a signal peptide (signal sequence trap (SST)) by using animal cells (See Japanese Published Patent Application No. Hei 6-315380). We also developed yeast SST method on the same conception. By the method based on the same conception using yeast (yeast SST method), genes encoding a signal peptide can be identified more easily and efficiently (See US Patent No. 5,536,637).

### Disclosure of the present invention

The present inventors have diligently performed certain investigation in order to isolate novel factors (polypeptides) useful for treatment, diagnosis and/or study, particularly, secretory proteins containing a secretory signal and membrane proteins.

From the result, using the above methods, the present inventors achieved to find novel secretory proteins and membrane proteins produced from cell lines and tissue, for example, human adult brain tissue, cell lines derived from human brain tissue, cell lines derived from human bone marrow, and human fetal liver, and cDNAs encoding them, and then completed the present invention.

The present invention provides cDNA sequences identified as clones OM007 and OMB096 which were isolated by the above yeast SST method using cDNA libraries prepared from human adult brain tissue. Clones OM007 and OMB096 were full-length cDNAs including full cDNA sequences encoding secretory proteins (represented as OM007 and OMB096 proteins, respectively).

It was indicated from the results of homology search for the public database of the nucleic acid sequences by using BLASTN and FASTA, and for the public database of the amino acid sequences by using BLASTX. BLASTP and FASTA, that there was no sequence identical to the polypeptide sequences of OM007 and OMB096 of the present invention and the nucleotide sequences encoding them. From these results, it was proved that the polypeptides of the present invention were new secretary proteins.

The present invention provides cDNA sequences identified as clones OAF0038-Leu and OAF038-Pro which were isolated by the above yeast SST method using cDNA libraries prepared from human adult born marrow (HAS303). Clones OAF0038-Leu and OAF038-Pro were full-length cDNAs including full cDNA sequences encoding membrane proteins (represented as OAF0038-Leu and OAF038-Pro proteins, respectively).

It was indicated from the results of homology search for the public database of the nucleic acid sequences by using BLASTN and FASTA, and for the public database of the amino acid sequences by using BLASTX. BLASTP and FASTA, that there was no sequence identical to the polypeptide sequences of OAF0038-Leu and OFA038-Pro of the present invention and the nucleotide sequences encoding them. From these results, it was proved that the polypeptides of the present invention were new membrane proteins.

The present invention provides a cDNA sequence identified as clone OR087H which was isolated by the above yeast SST method using cDNA libraries prepared from human fetal liver. Clone OR087H was a full-length cDNA including a full cDNA sequence encoding a secretory protein (represented as OR087H protein).

It was indicated from the results of homology search for the public database of the nucleic acid sequences by using BLASTN and FASTA, and for the public database of the amino acid sequences by using BLASTX, BLASTP and FASTA, that there was no sequence identical to the polypeptide sequence of OR087H of the present invention and the nucleotide sequences encoding it. From these results, it was proved that the polypeptide of the present invention was a new secretary protein.

The present invention provides cDNA sequences identified as clones OA004-FG and OA004-LD which were isolated by the above yeast SST method using cDNA libraries prepared from a human glioblastoma cell line T98G. Clones OA004-FG and OA004-LD were full-length cDNAs including full cDNA sequences encoding membrane proteins (represented as OA004-FG and OA004-LD proteins, respectively).

It was indicated from the results of homology search for the public database of the nucleic acid sequences by using BLASTN and FASTA, and for the public database of the amino acid sequences by using BLASTX, BLASTP and FASTA, that there was no sequence identical to the polypeptide sequences of OA004-FG and OA004-LD of the present invention and the nucleotide sequences encoding them. From these results, it was proved that polypeptides of the present invention were new membrane proteins.

That is to say, the present invention relates to:
(1) a polypeptide comprising the amino acid sequence of SEQ ID NOs: 1, 4, 7, 10, 13, 16 or 19,
(2) a cDNA encoding the polypeptide described in (1),
(3) a cDNA comprising the nucleotide sequence of SEQ ID NOs: 2, 5, 8, 11, 14, 17 or 20, and
(4) a cDNA comprising the nucleotide sequence of SEQ ID NOs: 3, 6, 9, 12, 15, 18 or 21.

### Detailed Description of the present invention

The present invention relates to a polypeptide in substantially purified form comprising the amino acid sequence shown in SEQ ID NOs: 1, 4, 7, 10, 13, 16 or 19, a homologue thereof, a fragment thereof, or a homologue of the fragment.

Further, the present invention relates to a cDNA encoding the above polypeptide. More particularly, the invention relates to a cDNA comprising the nucleotide sequence shown in SEQ ID NOs: 2, 5, 8, 11, 14, 17 or 20, and a cDNA containing a fragment which is selectively hybridized to a cDNA comprising the nucleotide sequence shown in SEQ ID NOs: 2, 3, 5, 6, 8, 9, 11, 12, 14, 15, 17, 18, 20 or 21. The cDNA capable of hybridizing to the cDNA includes the contemporary sequence of the above sequence. The conditions of the hybridizing are preferably stringent.

The polypeptide in substantially purified form comprising the amino acid sequence shown in SEQ ID NOs: 1, 4, 7, 10, 13, 16 or 19 will generally comprise the polypeptide in a preparation in which 90% or more, e.g., 95%, 98% or 99%, of the polypeptide in the preparation is that of the SEO ID NOs: 1, 4, 7, 10, 13, 16 or 19.

The homologue of the polypeptide comprising the amino acid sequence shown in SEQ ID NOs: 1, 4, 7, 10, 13, 16 or 19 will be generally at least 70%, preferably at least 80 or 90%, and more preferably at least 95%, homologous to the polypeptide over a region of at least 20, preferably at least 30, for instance 40, 60 or 100 or more, contiguous amino acids. Such a polypeptide homologue will be referred to as "a polypeptide of the present invention".

Also, the fragment of the polypeptide comprising the amino acid sequence shown in SEQ ID NOs: 1, 4, 7, 10, 13, 16 or 19 or its homologues will be generally at least 10, preferably at least 15, for example 20, 25, 30, 40, 50 or 60, amino acids in length.

The cDNA capable of selectively hybridizing to the cDNA comprising the nucleotide sequence shown in SEQ ID NOs: 2, 3, 5, 6, 8, 9, 11, 12, 14, 15, 17, 18, 20 or 21 will be generally at least 70%, preferably at least 80 or 90%, and more preferably at least 95%, homologous to the cDNA over a region of at least 20, preferably at least 30, for instance 40, 60 or 100 or more, contiguous nucleotides. Such a cDNA will be referred to as "a cDNA of the present invention".

The fragment of the cDNA comprising the nucleotide sequence shown in SEQ ID NO. 2, 3, 5, 6, 8, 9, 11, 12, 14, 15, 17, 18, 20 or 21 will be at least 10, preferably at least 15, for example 20, 25, 30 or 40, nucleotides in length. Such a fragment will be also referred to as "a cDNA of the present invention".

A further embodiment of the present invention provides replication and expression vectors carrying the cDNA of the present invention. The vectors may be, for example, plasmid, virus or phage vectors provided with an origin of replication, optionally a promoter for the expression of the cDNA and optionally a regulator of the promoter. The vectors may contain one or more selectable marker genes, for example an ampicillin resistance gene. The vectors may be used *in vitro,* for example, in the production of RNA corresponding to the cDNA, or the transformation of a host cell.

A further embodiment of the present invention provides host cells transformed with the vectors for the replication and expression of the cDNA of the present invention, including the cDNA comprising the nucleotide sequence shown in SEQ ID NOs: 2, 3, 5, 6, 8, 9, 11, 12, 14, 15, 17, 18, 20 or 21 or an open reading frame thereof. The cells include, for example, bacteria, yeast, insect cells, and mammalian cells.

A further embodiment of the present invention provides a method of producing a polypeptide which comprises culturing host cells of the present invention under conditions effective to express a polypeptide of the present invention. Preferably, in addition, such a method is carried out under conditions in which the polypeptide of the present invention is expressed and then produced from the host cells.

The cDNA of the present invention may also be inserted into the vectors described above in an antisense orientation in order to prove for the production of an antisense RNA. Such an antisense RNA may be used in a method of controlling the levels of the polypeptide of the present invention in a cell.

The present invention also provides monoclonal or polyclonal antibodies against the polypeptides of the present invention. The invention further provides a process for the production of monoclonal or polyclonal antibodies to the polypeptides of the present invention. The monoclonal antibodies may be prepared by common hybridoma technology using the polypeptides of the present invention or fragments thereof, as an immunogen. The polyclonal antibodies may also be prepared by common means which comprise inoculating host animals (for example, a rat, a rabbit, *etc*.) with the polypeptides of the present invention and recovering immune serum.

The present invention also provides pharmaceutical compositions comprising the polypeptide of the present invention or an antibody thereof, and a pharmaceutically acceptable diluent and/or carrier.

The polypeptides of the present invention specified in (1) include polypeptides in which a part of the amino acid sequence is lacking (e.g., a polypeptide comprised of only the essential sequence for revealing a biological activity from the amino acid sequence shown in SEQ ID NO: 1), polypeptides in which a part of their amino acid sequence is replaced by other amino acids (e.g., those replaced by an amino acid having a similar property), and polypeptides in which other amino acids are added or inserted into a part of their amino acid sequence, as well as those comprising the amino acid sequence shown in SEQ ID NOs: 1, 4, 7, 10, 13, 16 or 19.

As is well-known, there are one to six codons that encodes one amino acid (for example, one kind of codon for Met, and six codons for Leu). Accordingly, the nucleotide sequence of cDNA can be changed in order to encode a polypeptide having the same amino acid sequence.

The cDNAs of the present invention specified in (2) include a group of every nucleotide sequence encoding the polypeptide (1) shown in SEQ ID NOs: 1, 4, 7, 10, 13, 16 or 19. There is a probability that the yield of a polypeptide is improved by changing a nucleotide sequence.

The cDNAs specified by SEQ ID NOs: 2, 5, 8, 11, 14, 17 or 20 in (3) are the embodiment of the cDNAs shown in (2), and indicate the natural form of the sequence.

The cDNAs shown by SEQ ID NOs: 3, 6, 9, 12, 15, 18 or 21 in (4) indicates the sequence of the cDNAs specified in (3) with a natural non-translated region.

The cDNA having the nucleotide sequence shown in SEQ ID NOs: 3, 6, 9, 12, 15, 18, or 21 is prepared by the following method:

Brief description of Yeast SST method (see US Patent No. 5,536,637) is as follows.

Yeast, such as *Saccharomyces cerevisiae*, should secrete invertase into the medium in order to take sucrose or raffinose as a source of energy or carbon (the invertase is an enzyme to cleave raffinose into sucrose and melibiose, and sucrose into fructose and glucose). It is known that many known mammalian signal sequences make yeast secrete its invertase. From these knowledge, SST method was developed as a screening method to find a novel signal peptide which makes it possible to secrete yeast invertase from mammalian cDNA library using yeast growth on a raffinose medium as a marker.

Non-secretory type invertase gene SUC2 (GENBANK, Accession No. V01311) that lacks initiation codon ATG was inserted to a yeast expression vector to prepare vector pSUC2 for yeast SST. Into this expression vector, an expression promoter (ADH promoter) and a terminator (ADH terminator) both derived from AAH5 plasmid (Gammerer, Methods in Enzymol., 101, 192-201, 1983)) were inserted, and 2µ ori, TRP1, ColE1 ori and an ampicillin resistance gene were inserted as a yeast replication origin, a yeast selective marker, an *E. coli* replication origin and a drug resistance marker, respectively.

A mammalian cDNA was inserted into the upstream of SUC2 gene to prepare yeast SST cDNA library. This library was transformed into yeast that lacks secretory type invertase. If the inserted mammalian cDNA encodes a signal peptide, the yeast could survive in raffinose medium as a result of restoring secretion of invertase. Thus, it is possible to identify a novel signal peptide rapidly and easily by culturing expressed yeast colonies, preparing plasmids and determining the nucleotide sequence of the insert cDNAs,

Preparation of yeast SST cDNA library is as follows:
(1) mRNA is isolated from the targeted cells, double-strand synthesis is performed by using a random primer with a certain restriction enzyme (enzyme I) recognition site to obtain a double-strand cDNA,
(2) the obtained double-strand cDNA is ligated to an adapter containing a certain restriction endonuclease (enzyme II) recognition site, which is different from enzyme I, digested with enzyme I and fractionated to an appropriate size,
(3) the obtained cDNA fragment is inserted into a yeast expression vector on the upstream region of an invertase gene in which a signal peptide is deleted for transformation.

Detailed description of each step is as follows:
(1) mRNA is isolated from mammalian organs and cell lines stimulate them with an appropriate stimulator, if necessary) by known methods (Molecular Cloning (Sambrook, J., Fritsch, E. F. and Maniatis, T., Cold Spring Harbor Laboratory Press, 1989) or Current Protocol in Molecular Biology (F. M. Ausubel et al, John Wiley & Sons, Inc.) unless otherwise described hereinafter).

HAS303 (human bone marrow stroma cell line: provided from Professor Keisuke Sotoyama, Dr. Makoto Aizawa of First Medicine, Tokyo Medical College; see J. Cell. Physiol., 148, 245-251, 1991 and Experimental Hematol., 22, 482-487, 1994), human glioblastoma cell line TG98G (ATCC No. CRL-1690), and human fetal liver (CLONTECH, #CL6527-1) are chosen as a cell line. Human adult brain is chosen as a tissue source. Double-strand cDNA synthesis using random primer is performed by known methods.

Any sites may be used as restriction endonuclease recognition site I which is linked to an adapter and restriction endonuclease recognition site II which is used in step (2) if both sites are different from each other. Preferably, *Xho*I is used as enzyme I and *E*coRI as enzyme II.

In step (2), the cDNA is blunt-ended with T4 DNA polymerase, ligated to enzyme II adapter, digested with enzyme I, and fractionized to cDNAs of 300 to 800 bp with agarose-gel electrophoresis (AGE). As mentioned above, any enzyme may be used as enzyme II if it is different from enzyme I,

In step (3), cDNA fragments obtained in step (2) are inserted into a yeast expression plasmid vector on the upstream region of an invertase gene in which a signal peptide is deleted. *E. coli* was transformed with the expression vector. Many vectors are known as the yeast expression plasmid vector. For example, YEp24 which is also functioned in *E*. *coli* is used. Preferably, pSUC2 as described above is used.

Many host *E. coli* strains are known for transformation, preferably DH10B competent cell is used. Any known transformation method is available, preferably it is performed by electropolation method. The transformant is cultured by conventional methods to obtain cDNA library for yeast SST method.

However, the cDNA fragment is not inserted to all clones in this cDNA library. Further, all of the gene fragments do not encode unknown (novel) signal peptides. It is therefore necessary to screen a gene fragment encoding an unknown signal peptide from the library. Therefore, screening of fragments containing a sequence encoding a signal peptide is performed by transformation of the cDNA library into *Saccharomyces cerevisiae* having no invertase gene (e.g., YT455 strain) or a strain which artificially lacks an invertase gene (it may be prepared by known methods.).

Transformation of yeast is performed by known methods, e.g., lithium acetate method. A transformant is cultured in a selective medium, then transferred to a medium containing raffinose as a carbon source. Survival colonies are selected and then a plasmid is recovered. Survival colonies using raffinose as a carbon source indicates that some signal peptide of a secretory protein was inserted to this clone.

As for isolated positive clones, the nucleotide sequence is determined. As to a cDNA encoding an unknown protein, a full-length clone may be isolated by using the cDNA fragment as a probe and then the full-length nucleotide sequence was determined. These manipulations are performed by known methods.

Once the nucleotide sequences shown in SEQ ID NOs: 2, 5, 8, 11, 14, 17 or 20 are determined partially or preferably fully, it is possible to obtain cDNAs encoding the proteins of the present invention present in mammals or cDNAs encoding a homologue or subset of the proteins of the present invention. cDNA library or mRNA derived from mammals was screened by PCR with a synthesized oligonucleotide having an appropriate nucleotide sequence or by hybridization with a fragment having an appropriate nucleotide sequence as a probe. It is possible to obtain cDNAs encoding other mammalian homologue protein from other mammalian cDNA library or its genome library.

If the cDNA obtained above contains a nucleotide sequence of a cDNA fragment obtained by SST (or a consensus sequence thereof), it will be thought that the cDNA encodes the signal peptide. So it is clear that the cDNA will be full-length or almost full (all signal peptides exist at N-termini of a protein and are encoded at 5'-temini of the open reading frame of the cDNA).

The full-length may be confirmed by Northern analysis with the cDNA as a probe according to known methods. It is thought that the cDNA has an almost complete length, if the length of the cDNA is almost the same as the length of the mRNA obtained in the hybridizing band.

The proteins of the present invention include both a full-length type and a mature type. The full-length type and the mature type of the proteins are shown by SEQ ID NOs: 1, 4, 7, 10, 13, 16 and 19. These mature proteins can be obtained by expressing full-length cDNAs shown by SECT ID NOs: 3, 6, 9, 12, 15, 18 and 21 in appropriate mammalian cells or other host cells. The sequence of the matured protein can be predicted from the amino acid sequence of the full-length type.

Once the nucleotide sequence shown in SEQ ID NOs: 2, 5, 8, 11, 14, 17 or 20 is determined, the cDNAs of the present invention can be obtained by chemical synthesis, or by hybridization making use of nucleotide fragments which have been chemically synthesized as a probe. Furthermore, the cDNAs of the present invention can be obtained in a desired amount by transforming a vector cDNA that contains the cDNA into a proper host, and culturing the transformant.

The polypeptides of the present invention may be prepared by, for example:
(1) isolating and purifying such polypeptides from an organism or a cultured cell,
(2) chemical peptide synthesis, or
(3) using recombinant cDNA technology,
preferably, by the method described in (3) in an industrial production.

Examples of expression system (host-vector system) for producing a polypeptide by using recombinant cDNA technology are the expression systems of bacteria, yeast, insect cells and mammalian cells.

In the expression, for example, in *E*. *coli*, the expression vector is prepared by adding the initiation codon (ATG) to 5'-end of a cDNA encoding a mature protein, connecting the cDNA thus obtained to the downstream of a proper promoter (e.g., *trp* promoter, *lac* promoter, λPL promoter, T7 promoter *etc*.), and then inserting it into a vector (e.g., pBR322, pUC18, pUC19 *etc*.) which functions in an *E*. *coli* strain.

Then, an *E*. *coli* strain (e.g., *E*. *coli* DH1 strain, *E*. *coli* JM109 strain, *E. coli* HB101 strain, *etc*.) which is transformed with the expression vector described above may be cultured in an appropriate medium to obtain the desired polypeptide. When a signal sequence of bacteria (e.g., signal sequence of pel B) is utilized, the desired polypeptide may also be released in periplasm. Furthermore, a fusion protein with other polypeptide may also be produced.

In the expression of the polypeptides in mammalian cells, for example, the expression vector is prepared by inserting the cDNA encoding the nucleotide shown in SEQ ID NOs: 2, 5, 8, 11, 14, 17 or 20 into the downstream of a proper promoter (e.g., SV40 promoter, LTR promoter, metallothionein promoter, *etc*.) in a proper vector (e.g., retrovirus vector, papilloma virus vector, vaccinia virus vector, SV40 vector, *etc*.). A proper mammalian cell (e.g., monkey COS-7 cell, Chinese hamster CHO cell, mouse L cell, *etc*.) is transformed with the expression vector thus obtained, and then the transformant is cultured in a proper medium to express the protein (polypeptide) of the present invention by the following method depending on whether it is a secretory protein or a membrane protein.

In case of a secretory protein as the protein of the present invention, the aimed polypeptide was expressed in the supernatant of the cells. In addition, a fusion protein may be prepared by conjugating a cDNA fragment encoding other polypeptide, for example, the Fc portion of an antibody.

On the other hand, in case of a membrane protein as the protein of the present invention, the aimed polypeptide was expressed on the cell membrane. A cDNA encoding the nucleotide sequence of SEQ ID NO. 9, 12, 18 or 21 with deletion of the extracellular region was inserted into the above vector, transfected into the adequate mammalian cells to secret the aimed soluble polypeptide in the culture medium. In addition, a fusion protein may be prepared by conjugating a cDNA fragment encoding the mutant with deletion of the extracellular region and other polypeptide, for example, the Fc portion of an antibody.

The polypeptide available by the way described above can be isolated and purified by conventional biochemical method.

### Industrial Applicability

It is considered that the polypeptides of the present invention and cDNAs encoding them will show one or more effects or biological activities (including those which relates to assays cited below). The effects or biological activities described in relation to the proteins of the present invention are provided by administration or use of the protein or by administration or use of a cDNA molecule which encodes the protein (e.g., vector suitable for gene therapy or cDNA introduction).

### Cytokine activity and cell proliferation/differentiation activity:

The protein of the present invention may exhibit cytokine activity, cell proliferation (either inducing or inhibiting) or cell differentiation (either inducing or inhibiting) activity, or may induce production of other cytokines in certain cell populations. Many protein factors discovered to date, including all known cytokines, have exhibited activity in one or more factor dependent cell proliferation assays, and hence the assays serve as a convenient confirmation of cytokine activity. The activity of the polypeptide of the present invention is evidenced by any one of a number of routine factor dependent cell proliferation assays for cell lines.

### Immune stimulating/suppressing activity:

The protein of the present invention may also exhibit immune stimulating or immune suppressing activity. The protein of the present invention may be useful in the treatment of various immune deficiencies and disorders (including severe combined immunodeficiency (SCID)), e.g., in regulating (up or down) growth and proliferation of T and/or B lymphocytes, as well as effecting the cytolytic activity of NK cells and other cell populations. These immune deficiencies may be genetic or be caused by viral infection, such as HIV, as well as bacterial or fungal infections, or may result from autoimmune disorders. More specifically, infectious diseases causes by viral, bacterial, fungal or other infection may be treatable using the protein of the present invention, including infections by HIV, hepatitis viruses, herpes viruses, mycobacteria, leshmania, malaria, and various fungal infections, such as candida. Of course, in this regard, the protein of the present invention may also be useful where a boost to the immune system generally would be indicated, i.e., in the treatment of a cancer.

The protein of the present invention may be useful in the treatment of allergic reactions and conditions, such as asthma or other respiratory problems. The protein of the present invention may also be useful in the treatment of the other conditions required to suppress the immuno system (for example, asthma or respiratory disease).

The protein of the present invention may also suppress chronic or acute inflammation, for example, that associated with infection, such as septic shock or systemic inflammatory response syndrome (SIRS), inflammatory bowel disease, Crohn's disease, or resulting from over production of cytokines, such as TNF or IL-1, wherein the effect was demonstrated by IL- 11.

### Hematopoiesis regulating activity:

The protein of the present invention may be useful in regulation of hematopoiesis and, consequently, in the treatment of myeloid or lymphoid cell deficiencies. Even marginal biological activity in support of colony forming cells or of factor-dependent cell lines indicates involvement in regulating hematopoiesis. The biological activities are concerned with the following all or some example(s), e.g., in supporting the growth and proliferation of erythroid progenitor cells alone or in combination with other cytokines, thereby indicating utility, for example, in treating various anemia or for use in conjunction with irradiation/chemotherapy to stimulate the production of erythroid precursors and/or erythroid cells; in supporting the growth and proliferation of myeloid cells, such as granulocytes and monocytes/macrophages, (i.e., traditional CSF activity) useful, for example, in conjunction with chemotherapy to prevent or treat consequent myelo-suppression; in supporting the growth and proliferation of megakaryocytes and consequently of platelets thereby allowing prevention or treatment of various platelet disorders, such as thrombocytopenia, and generally for use in place of or complimentary to platelet transfusions; and/or in supporting the growth and proliferation of hematopoietic stem cells which are capable of maturing to any and all of the above-mentioned hematopoietic cells and therefore find therapeutic utility in various stem cell disorders (such as those usually treated with transplantation, including, without limitation, aplastic anemia and paroxysmal nocturnal hemoglobinuria), as well as in repopulating the stem cell compartment post irradiation/chemotherapy, either *in vitro* or *ex vivo* (i.e., in conjunction with bone marrow transplantation) as normal cells or genetically manipulated for gene therapy.

The activity of the protein of the present invention may, among other means, be measured by the following methods :

### Tissue generation/regeneration activity:

The protein of the present invention may also have utility in compositions used for bone, cartilage, tendon, ligament and/or nerve tissue growth or regeneration, as well as for wound healing and tissue repair, and in the treatment of bums, incisions and ulcers.

The protein of the present invention, which induces cartilage and/or bone growth in circumstances where bone is not normally formed, may be applied to the healing of bone fractures and cartilage damage or defects in humans and other animals. Such a preparation employing the protein of the present invention may have prophylactic use in closed as well as open fracture reduction and also in the improved fixation of artificial joints. *De novo* bone formation induced by an osteogenic agent contributes to the repair of congenital, trauma induced, or oncologic resection induced craniofacial defects, and also is useful in cosmetic plastic surgery.

The protein of the present invention may also be used in the treatment of periodontal disease, and in other tooth repair processes. Such agents may provide an environment to attract bone-forming cells, stimulate growth of bone-forming cells or induce differentiation of progenitors of bone-forming cells. The protein of the present invention may also be useful in the treatment of osteoporosis or osteoarthritis, such as through stimulation of bone and/or cartilage repair or by blocking inflammation or processes of tissue destruction (collagenase activity, osteoclast activity, *etc*.) mediated by inflammatory processes.

Another category of tissue regeneration activity that may be attributable to the protein of the present invention is tendon/ligament formation. The protein of the present invention, which induces tendon/ligament-like tissue or other tissue formation in circumstances where such tissue is not normally formed, may be applied to the healing of tendon or ligament tears, deformities and other tendon or ligament defects in humans and other animals. Such a preparation employing the protein inducing a tendon/ligament-like tissue may have prophylactic use in preventing damage to tendon or ligament tissue, as well as use in the improved fixation of tendon or ligament to bone or other tissues, and in repairing defects to tendon or ligament tissue. *De novo* tendon/ligament-like tissue formation induced by a composition of the present invention contributes to the repair of congenital, trauma induced, or other tendon or ligament defects of other origin, and is also useful in cosmetic plastic surgery for attachment or repair of tendons or ligaments. The compositions of the present invention may provide an environment to attract tendon- or ligament-forming cells, stimulate growth of tendon- or ligament-forming cells, induce differentiation of progenitors of tendon- or ligament-forming cells, or induce growth of tendon Ligament cells or progenitors *ex vivo* for return *in vitro* (*in vivo*) to effect tissue repair. The compositions of the present invention may also be useful in the treatment of tendinitis, Carpal tunnel syndrome and other tendon or ligament defects. The compositions may also include an appropriate matrix and a sequestering agent which is well known in the art as well as a carrier.

The protein of the present invention may also be useful for proliferation of neural cells and for regeneration of nerve and brain tissue. i.e. for the treatment of central and peripheral nervous system diseases and neuropathies. as well as mechanical and traumatic disorders, which involve degeneration, death or trauma to neural cells or nerve tissue. More specifically, the protein of the present invention may be used in the treatment of diseases of the peripheral nervous system, such as peripheral nerve injuries, peripheral neuropathy and localized neuropathies, and central nervous system diseases, such as Alzheimer's, Parkinson's disease, Huntington's disease, amyotrophic lateral sclerosis, and Shy-Drager syndrome. Further conditions which may be treated in accordance with the invention include mechanical and traumatic disorders, such as spinal cord disorders, head trauma and cerebrovascular diseases such as stroke. Peripheral neuropathies resulting from chemotherapy or other medical therapies may also be treatable using the protein of the present invention.

It is expected that the protein of the present invention may also exhibit activity for generation of other tissues, such as organs (including, for example, pancreas, liver, intestine, kidney, skin, endothelium), muscle (smooth, skeletal or cardiac) and vascular (including vascular endothelium) tissue, or for promoting the proliferation of cells comprising such tissues. A part of the desired effects may be by inhibition of fibrotic scarring to allow normal tissue to regenerate.

The protein of the present invention may also be useful for gut protection or regeneration and treatment of lung or liver fibrosis, reperfusion injury in various tissues, and conditions resulting from systemic cytokine damage.

### Activin/Inhibin activity:

The protein of the present invention may also exhibit activin- or inhibin-related activities. Inhibins are characterized by their ability to inhibit the release of follicle stimulating hormone (FSH), while activins are characterized by their ability to stimulate the release of follicle stimulating hormone (FSH). Thus, the protein of the present invention alone or in heterodimers with a member of the inhibin α family, may be useful as a contraceptive based on the ability of inhibins to decrease fertility in female mammals and decrease spermatogenesis in male mammals. Administration of sufficient amounts of other inhibins can induce infertility in these mammals. Alternatively, the protein of the present invention, as a homodimer or as a heterodimer with other protein subunits of the inhibin-β group, may be useful as a fertility inducing therapeutic, based upon the ability of activin molecules in stimulating FSH release from cells of the anterior pituitary (See US Patent 4,798,885).
The protein of the present invention may also be useful for advancement of the onset of fertility in sexually immature mammals, so as to increase the lifetime reproductive performance of domestic animals such as cows, sheep and pigs.

### Chemotactic/chemokinetic activity:

The protein of the present invention may have chemotactic or chemokinetic activity, e.g., functioning as a chemokine, for mammalian cells, including, for example, monocytes, neutrophils, T-cells, mast cells, eosinophils and/or endothelial cells. Chemotactic and chemokinetic proteins can be used to mobilize or attract a desired cell population to a desired site of action. Chemotactic or chemokinetic proteins provide particular advantages in treatment of wounds and other trauma to tissues, as well as in treatment of localized infections. For example, attraction of lymphocytes, monocytes or neutrophils to tumors or sites of infection may result in improved immune responses against the tumor or infecting site.

If a protein or peptide can stimulate, directly or indirectly, the directed orientation or movement of such cell population, it has chemotactic activity for a particular cell population. Preferably, the protein or peptide has the ability to directly stimulate directed movement of cells. Whether a particular protein has chemotactic activity for a population of cells can be readily determined by employing such protein or peptide in any known assay for cell chemotaxis.

### Hemostatic and thrombolytic activity:

The protein of the present invention may also exhibit hemostatic or thrombolyic activity. As a result, such a protein is expected to be useful in treatment of various coagulation disorders (including hereditary disorders, such as hemophilias) or to enhance coagulation and other hemostatic events in treating wounds resulting from trauma, surgery or other causes. The protein of the present invention may also be useful for dissolving or inhibiting formation of thromboses and for treatment and prevention of conditions resulting from, for example, infarction, stroke, *etc*.

### Receptor/ligand activity:

The protein of the present invention may also demonstrate activity as receptors, receptor ligands or inhibitors or agonists of receptor/ligand interactions. Examples of such receptors and ligands include, without limitation, cytokine receptors and their ligands, receptor kinases and their ligands, receptor phosphatases and their ligands, receptors involved in cell-cell interactions and their ligands (including cellular adhesion molecules such as Selectins, Integrins and their ligands) and receptor/ligand pairs involved in antigen presentation, antigen recognition and development of cellular and humoral immune responses. Receptors and ligands are also useful for screening of potential peptide or small molecule inhibitors of the relevant receptor/ligand interaction. The protein of the present invention (including, without limitation, fragments of receptors and ligands) *per se* may be useful as inhibitors of receptor/ligand interactions.

### Other activity:

The protein (polypeptide) of the present invention may also exhibit one or more of the following additional activities or effects: inhibiting growth of or killing the infecting agents including bacteria, viruses, fungi and other parasites; suppressing or enhancing body characteristics including height, weight, hair color, eye color, skin, other tissue pigmentation, size of organs, for example, breast augmentation, diminution *etc*.; effecting elimination of dietary fat, protein, carbohydrate; effecting behavioral characteristics including appetite, libido, stress, cognition (including cognitive disorders), depression and violent behaviors; providing analgesic effects or other pain reducing effects; promoting differentiation and growth of embryonic stem cells in lineages other than hematopoietic lineages; in the case of enzymes, correcting deficiencies of the enzyme and treating deficiency-related diseases.

The protein with above activities, is suspected to have following functions by itself or interaction with its ligands or receptors or association with other molecules. For example, proliferation or cell death of B cells, T cells and/or mast cells; specific induction by promotion of class switch of immunoglobulin genes; differentiation of B cells to antibody-forming cells; proliferation, differentiation, or cell death of precursors of granulocytes; proliferation, differentiation, or cell death of precursors of monocytes-macrophages; proliferation, of up regulation or cell death of neutrophils, monocytes-macrophages, eosinophils and/or basophils; proliferation, or cell death of precursors of megakaryocytes; proliferation, differentiation, or cell death of precursors of neutrophils; proliferation, differentiation, or cell death of precursors of T cells and B cells; promotion of production of erythrocytes; sustainment of proliferation of erythrocytes, neutrophils, eosinophils, basophils, monocytes-macrophages, mast cells, precursors of megakaryocyte; promotion of migration of neutrophils, manocytes-macrophages, B cells and/or T cells; proliferation or cell death of thymocytes; suppression of differentiation of adipocytes; proliferation or cell death of natural killer cells; proliferation or cell death of hematopoietic stem cells; suppression of proliferation of stem cells and each hematopoietic precursor cells; promotion of differentiation from mesenchymal stem cells to osteoblasts or chondrocytes, proliferation or cell death of mesenchymal stem cells, osteoblasts or chondrocytes and promotion of bone absorption by activation of osteoclasts and promotion of differentiation from monocytes to osteoclasts.

The polypeptide of the present invention is also suspected to function to nervous system, so expected to have functions below; differentiation to kinds of neurotransmitter-responsive neurons, survival or cell death of these cells; promotion of proliferation or cell death of glial cells; spread of neural dendrites; survival or cell death of gangriocytes; proliferation, promotion of differentiation, or cell death of astrocytes; proliferation, survival or cell death of peripheral neurons; proliferation or cell death of Schwann cells; proliferation, survival or cell death of motoneurons.

Furthermore, in the process of development of early embryonic, the polypeptide of the present invention is expected to promote or inhibit the organogenesis of epidermis, brain, backbone, and nervous system by induction of ectoderm, that of notochord connective tissues (bone, muscle, tendon), hemocytes, heart, kidney, and genital organs by induction of mesoderm, and that of digestive apparatus (stomach, intestine, liver, pancreas), respiratory apparatus (lung, trachea) by induction of endoderm. In adult, also, this polypeptide is thought to proliferate or inhibit the above organs.

Therefore, the polypeptide of the present invention itself is expected to be used as an agent for the prevention or treatment of disease of progression or suppression of immune, nervous, or bone metabolic function, hypoplasia or overgrowth of hematopoietic cells: for example, inflammatory disease (rheumatism, ulcerative colitis, *etc*.), decrease of hematopoietic stem cells after bone marrow transplantation, decrease of leukocytes, platelets, B-cells, or T-cells after radiation exposure or chemotherapeutic dosage against cancer or leukemia, anemia, infectious disease, cancer, leukemia, AIDS, bone metabolic disease (osteoporosis *etc*.), various degenerative disease (Alzheimer's disease, multiple sclerosis, *etc*.), or nervous lesion.

In addition, since the polypeptide of the present invention is thought to induce the differentiation or growth of organs derived from ectoderm, mesoderm, and endoderm, this polypeptide is expected to be an agent for tissue repair (epidermis, bone, muscle, tendon, heart, kidney, stomach, intestine, liver, pancreas, lung, trachea, *etc*.).

By using polyclonal or monoclonal antibodies against the polypeptide of the present invention, quantitation of the polypeptide in the body can be performed. It can be used in the study of relationship between this polypeptide and disease or diagnosis of disease, and so on. Polyclonal and monoclonal antibodies can be prepared using the polypeptide or its fragment as an antigen by conventional methods.

Identification, purification or molecular cloning of known or unknown proteins which bind the polypeptide of the present invention (preferably polypeptide of extracellular domain) can be performed using the polypeptide of the present invention by, for example, preparation of the affinity-column.

Identification of the downstream signal transmission molecules which interact with the polypeptide of the present invention in cytoplasma and molecular cloning of the gene can be performed by west-western method using the polypeptide of the present invention (preferably polypeptide of transmembrane region or intracellular domain), or by yeast two-hybrid system using the cDNA (preferably cDNA encoding the transmembrane region or cytoplasmic domain of the polypeptide).

Agonists/antagonists of this receptor polypeptide and inhibitors between receptor and signal transduction molecules can be screened using the polypeptide of the present invention.

The cDNAs of the present invention are useful not only the important and essential template for the production of the polypeptide of the present invention which is expected to be largely useful, but also be useful, for diagnosis or therapy (for example, treatment of gene lacking, treatment to stop the expression of the polypeptide by antisense cDNA (mRNA)). Genomic cDNA may be isolated wish the cDNA of the present invention, as a probe. In the same manner, a human gene encoding a related polypeptide which can be highly homologous to the cDNA of the present invention or gene encoding a polypeptide highly homologous to the polypeptide of the present invention and a gene of animals excluding mouse encoding a polypeptide which can be highly homologous to the polypeptide of the present invention, also may be isolated.

### Application to Medicaments:

The polypeptide of the present invention or the antibody specific for the polypeptide of the present invention is administered systemically or topically and in general orally or parenterally, preferably parenterally, intravenously and intraventricularly, for preventing or treating the diseases.

The doses to be administered depend upon age, body weight, symptom, desired therapeutic effect, route of administration, and duration of the treatment *etc*. In human adults, one dose per person is generally between 100 µg and 100 mg, by oral administration, up to several times per day, and between 10 µg and 100 mg, by parental administration, up to several times per day.

As mentioned above, the doses to be used depend upon various conditions. Therefore, there are cases in which doses lower than or greater than the ranges specified above may be used.

The compounds of the present invention may be administered as solid compositions, liquid compositions or other compositions for oral administration, as injections, liniments or suppositories *etc*. for parental administration.

Solid compositions for oral administration include compressed tablets, pills, capsules, dispersible powders, and granules. Capsules include soft and hard capsules.

In such compositions, one or more of the active compound(s) may be admixed with at least one inert diluent (such as lactose, mannitol, glucose, hydroxypropyl cellulose, microcrystalline cellulose, starch, polyvinylpyrrolidone, magnesium metasilicate aluminate, *etc*.). The compositions may also comprise, as is normal practice, additional substances other than inert diluents: e.g., lubricating agents (such as magnesium stearate *etc*.), disintegrating agents (such as cellulose calcium glycolate, *etc*.), stabilizing agents (such as human serum albumin, lactose *etc*.), and assisting agents for dissolving (such as arginine, asparaginic acid *etc*.).

The tablets or pills may, if desired, be coated with a film of gastric or enteric materials (such as sugar, gelatin, hydroxypropyl cellulose, hydroxypropylmethyl cellulose phthalate, *etc*.), or be coated with at least two films. And then, coating may include containment within capsules of absorbable materials such as gelatin.

Liquid compositions for oral administration include pharmaceutically-acceptable emulsions, solutions, syrups and elixirs. In such compositions, one or more of the active compound(s) may be contained in inert diluent(s) commonly used (purified water, ethanol *etc*.). Besides inert diluents, such compositions may also comprise adjuvants (such as wetting agents, suspending agents, *etc*.), sweetening agents, flavoring agents, perfuming agents, and preserving agents.

Other compositions for oral administration include spray compositions which may be prepared by known methods and which comprise one or more of the active compound(s). Spray compositions may comprise additional substances other than inert diluents: e.g., stabilizing agents (sodium sulfite *etc*.), isotonic buffer (sodium chloride, sodium citrate, citric acid, *etc*.). For preparation of such spray compositions, for example, the method described in the US Patent No. 2,868,691 or 3,095,355 may be used.

Injections for parental administration include sterile aqueous or non-aqueous solutions, suspensions and emulsions. In such compositions, one or more active compound(s) is or are admixed with at least one inert aqueous diluent(s) (distilled water for injection, physiological salt solution, *etc*.) or inert non-aqueous diluents(s)(propylene glycol, polyethylene glycol, olive oil, ethanol, POLYSOLBATE 80 (Trademark) *etc*.).

Injections may comprise additional compound other than inert diluents:
e.g., preserving agents, wetting agents, emulsifying agents, dispersing agents, stabilizing agent (such as human serum albumin, lactose, *etc*.), and assisting agents, such as assisting agents for dissolving (arginine, asparaginic acid, *etc*.).

### Best Mode carrying out the Invention

The invention is illustrated by the following example, but the invention is not limited thereto.

### Example 1: Clone OM007

### Preparation of Poly(A)⁺RNA:

A total RNA was prepared from human adult brain tissue by TRlzol reagent (Trademark, marketed by GIBCO BRL Co.). Poly(A)⁺RNA was purified from the total RNA by mRNA Purification Kit (Trade name, marketed by Pharmacia Co.).

### Preparation of yeast SST cDNA library:

A double strand cDNA was synthesized by Super Script Plasmid System for cDNA Synthesis and Plasmid Cloning (Trade name, marketed by GIBCOBRL Co.) with above poly(A)⁺RNA as template and random 9mer as primer which was containing Xhol site:
5'-CGATTGAATTCTAGACCTGCCTCGAGNNNNNNNNN-3 (SEO ID NO: 22).
The cDNA was ligated with *E*coRI adapter by DNA ligation kit ver. 2 (Trade name, marketed by Takara Shuzo Co.; this kit was used in all ligating steps hereinafter.) and digested by *Xho*I*.* The cDNAs were separated by agarose-gel electrophoresis. 300 to 800 bp cDNAs were isolated and were ligated to *Eco*RI/*Not*I site of pSUC2 (see US Patent No. 5,536,637). *E. coli* DH10B strains were transformed by pSUC2 with electropolation to obtain yeast SST cDNA library.

### Screening by SST method and determination of nucleotide sequence of SST positive clone:

Plasmid of the cDNA library were prepared. Yeast YTK12 strains were transformed by the plasmids with lithium acetate method (*Current Protocols In Molecular Biology,* 13.7.1). The transformed yeast were plated on a triptphan-free medium (CMD-Trp medium) for selection. The plate was incubated for 48 hour at 30°C. Replica of the colony (transformant) which was obtained by Accutran Replica Plater (Trade name, marketed by Schleicher & Schuell) were placed onto YPR plate containing raffinose for a carbon source, and the plate was incubated for 14 days at 30°C. After 3 days, each colony appeared was streaked on YPR plate again. The plates were incubated for 48 hours at 30°C. Single colony was inoculated to YPD medium and was incubated for 48 hours at 30°C. Then plasmids were prepared. An insert cDNA was amplified by PCR with two kind primers which exist on the end side of the cloning site on pSUC2 (sense strand primers were biotinylated). Biotinylated single strand of cDNAs were purified with Dynabeads (Trade name, marketed by DYNAL Co.) and the nucleotide sequences were determined. Sequencing was performed by Dye Terminator Cycle Sequencing Ready Reaction with DNA Sequencing kit (Trade name, marketed by Applied Biosystems Inc.) and the sequence was determined by DNA sequencer 373 (Applied Biosystems Inc.) (all sequencing hereafter was carried out with this method.).

We tried to carry out cloning of full-length cDNA which was proved to be new one according to the homology search for the obtained nucleotide sequences and deduced amino acid sequences in data base. We also confirmed that each cDNA contains a signal peptide in view of function and structure, by comparison with a known signal peptide and the deduced amino acid sequence.

### Cloning of a full-length cDNA and determination of nucleotide sequence:

A full-length cDNA was cloned using Marathon cDNA Amplification Kit (Trade name, marketed by Clontech Co.) according to 3' RACE (Rapid Amplification of cDNA End) method. A double strand cDNA was prepared from the origin of each clone, i.e., poly(A)⁺RNA in human adult brain tissue. 27mer primer OM007-F3:
5'-AACTGCAGATCTTGGGACTCATCAGCC-3' (SEQ ID NO: 23)
containing the deduced initiation ATG codon region based on the information of the nucleotide sequence obtained by SST, was prepared. PCR was performed with the primer and an adapter primer attached in the kit. Due to insufficient amplification of cDNA by only one-time PCR, 28mer primer OM007-F2:
5'-AAGAGGACATTGTTTTCATGATGGATGC-3' (SEQ ID NO: 24)
was prepared additionally at 3'-end of OM007-F1 primer and then nested PCR was performed. A cDNA which was amplified with clone OM007 specifically was separated with agarose-gel electrophoresis, ligated to pT7 Blue-2 T-Vector (Trade name, marketed by Novagen Co.) and transfected into *E*. *coli* DH5a to prepare a plasmid. First, nucleotide sequences of 5'-end were determined, and the existence of nucleotide sequence OM007 SST cDNA was confirmed. A nucleotide sequence of full-length OM007 SST cDNA was determined and then the cDNA sequence shown in SEQ ID NO: 3 was obtained. An open reading frame was determined and deduced an amino acid sequence, and sequences shown in SEQ ID NOs: 1 and 2 were obtained.

It was indicated from the results of homology search for the public database of the nucleic acid sequences by using BLASTN and FASTA, and for the public database of the amino acid sequences by using BLASTX, BLASTP and FASTA, that there was no sequence identical to the polypeptide sequence of OM007 of the present invention and the nucleotide sequences encoding them. From these results, it was proved that polypeptide of the present invention was a new secretary protein. However, the search using BLASTX, BLASTP and FASTA revealed a significant homology between clone OM007 (region of 21st to 765th amino acid in SEQ ID NO; 1) and a region of 9th to 753rd amino acids chicken collapsin-2 (collapsin-2, (Gallus galius), Genbank Accession U28240). Based on the homology, clone OM007 and semaphorin family to which collapsin belongs are expected to share at least some activity.

### Example 2: Clone OMB096

In Example relating to clone OMB096 of the present invention, the same procedure as in Example of OM007 was used, except for the following points.

### Cloning of a full-length cDNA and determination of nucleotide sequence:

A full-length cDNA was cloned using Marathon cDNA Amplification Kit (Trade name, marketed by Clontech Co.) according to 3' RACE method in the same manner as in Example of OM007. A double strand cDNA was prepared from the origin of each clone, i.e., poly(A)⁺RNA in human adult brain tissue. 27mer primer OMB096-F1:
5'-ACAACATGCACCACCAGTGGCTTCTGC-3' (SEQ ID NO: 25)
containing the deduced initiation ATG codon region based on the information of the nucleotide sequence obtained by SST, was prepared. PCR was performed with the primer and an adapter primer attached in the kit. A cDNA which was amplified with clone OMB096 specifically was cloned in the same manner as in Example of OM007, a full nucleotide sequence was determined and then a cDNA sequence shown in SEQ ID NO: 6 was obtained. An open reading frame was determined and deduced an amino acid sequence, and sequences shown in SEQ ID NOs: 4 and 5 were obtained.

It was indicated from the results of homology search for the public database of the nucleic acid sequences by using BLASTN and FASTA, and for the public database of the amino acid sequences by using BLASTX, BLASTP and FASTA, that there was no sequence identical to the polypeptide sequence OMB096 of the present invention and the nucleotide sequences encoding it. From these results, it was proved that polypeptide of the present invention was a new secretary protein.

### Example 3: Clones OAF038-Leu and OAF038-Pro

In Example relating to clones OAF038-Leu and OAF038-Pro of the present invention, the same procedure as in Example of OM007 was used, except for the following points.

### Preparation of Poly(A)⁺RNA:

A total RNA was prepared from human bone marrow stroma cell line HAS303 (provided from Prof. Keisuke Sotoyama, Dr. Makoto Aizawa, First Medicine, Tokyo Medical College) by TRlzol reagent (Trademark, marketed by GIBCOBRL Co.). Poly(A)⁺RNA was purified from the total RNA by mRNA Purification Kit (Trade name, marketed by Pharmacia Co.).

### Cloning of a full-length cDNA and determination of nucleotide sequence:

A full-length cDNA was cloned using Marathon cDNA Amplification Kit (Trade name, marketed by Clontech Co.) according to 3' RACE method in the same manner as in Example of OM007. Double strand cDNA was prepared from the origin of each clone, i.e., poly(A)⁺RNA in HAS303. 28mer primer OAF038-F1:
5'-AGAATGTGGAGCCATTTGAACAGGCTCC-3' (SEQ ID NO, 26)
containing the deduced initiation ATG codon region based on the information of the nucleotide sequence obtained by SST, was prepared. PCR was performed with the primer and an adapter primer attached in the kit. A cDNA which was amplified with clone OAF038 specifically was separated with recloning by the same method as in Examples of OM007, a full-length nucleotide sequence was determined, and then cDNA sequences shown in SEQ ID NOs: 9 and 12 were obtained. Each clone was named OAF038-Leu and OAF308-Pro, respectively. Open reading frames were determined and deduced amino acid sequences, and sequences shown in SEQ ID NOs: 7 and 8 and NOs: 10 and 11, respectively, were obtained.

It was indicated from the results of homology search for the public database of the nucleic acid sequences by using BLASTN and FASTA, and for the public database of the amino acid sequences by using BLASTX, BLASTP and FASTA, that there was no sequence identical to the polypeptide sequences of OAF038-Leu and OAF038-Pro of the present invention and the nucleotide sequences encoding them. From these results, it was proved that polypeptides of the present invention were new membrane proteins. However, the search using BLASTX, BLASTP and FASTA revealed a significant homology between clones OAF038-Leu and OAF038-Pro (region of 5th to 343rd amino acids in SEQ ID NOs: 7 and 10) and rat MCA-32 protein (Rat MCA-32 protein, Genbank Accession U39546, a region of 42nd to 268th amino acids). Polypeptides OAF038-Leu and OAF038-Pro have Ig domain and SH2 domain at the extracell domain and the cytoplasmic domain, respectively, in the same manner as Rat MCA-32 protein. Based on the homology, clones OAF038-Leu and OAF038-Pro and the above Rat MCA-32 protein are expected to share at least some activity.

### Example 4: Clone OR087H

In Example relating to clone OR087H of the present invention, the same procedure as in Example of OM007 was used, except for the following points.

### Preparation of Poly(A)⁺RNA:

We purchased poly(A)⁺RNA (CL6527-1) in human fetal liver from CLONTECH Co.

### Cloning of a full-length cDNA and determination of nucleotide sequence:

A full-length cDNA was cloned using Marathon cDNA Amplification Kit (Trade name, marketed by Clontech Co.) according to 3' RACE method in the same manner as in Example of OM007. A double strand cDNA conjugating an adapter was prepared from the origin of each clone, i.e., paly(A)⁺RNA in human fetal liver according to the method of the kit. 27mer primer OR087H-F1:
5'-TGAAGCCCTTGTCCGTAAGCCTTGAAC-3' (SEQ ID NO: 27)
containing the deduced initiation ATG codon region based on the information of nucleotide sequence obtained by SST, was prepared. PCR was performed with the primer and an adapter primer attached in the kit. A cDNA which was amplified with clone OR087H specifically was separated with recloning by the same method as in Example of OM007. A full-length nucleotide sequence was determined and then cDNA sequence shown in SEQ ID NO: 15 was obtained. An open reading frame was determined and deduced an amino acid sequence and sequences shown in SEQ ID NOs: 13 and 14 were obtained.

It was indicated from the results of homology search for the public database of the nucleic acid sequences by using BLASTN and FASTA, and for the public database of the amino acid sequences by using BLASTX, BLASTS and FASTA, that there was no sequence identical to the polypeptide sequence of OR087H of the present invention and the nucleotide sequences encoding them. From these results, it was proved that polypeptide of the present invention was a new secretary protein. However, the search using BLASTX, BLASTP and FASTA revealed a significant homology between clone OR087H (region of 1st to 115th amino acids in SEQ ID NO: 13) and human rapamycin-and FK506-binding protein (rapamycin- and FK506-binding protein (Homo sapiens), Genbank Accession M75099, a region of 1st to 116th amino acids). Based on the homology, clone OR087H and FK-binding protein family are expected to share at least some activity.

### Example 5: Clones OA004-FG and OA004-LD

In Example relating to clones OA004-FG and OA004-LD of the present invention, the same procedure as in Example of OM007 was used, except for the following points.

### Preparation of Poly(A)⁺RNA:

Total RNA was prepared from human glioblastoma cell line T98G (ATCC No. CRL-1690) by TRlzol reagent (Trademark, marketed by GIBCOBRL Co.). Poly(A)⁺RNA was purified from the total RNA by mRNA Purification Kit (Trade name, marketed by Pharmacia Co.).

### Cloning of a full-length cDNA and determination of nucleotide sequence:

A full-length cDNA was cloned using GENETRAPPER cDNA Positive Selection System (Trade name, marketed by GIBCOBRL). First, dT-primed cDNA library was prepared from poly(A)⁺RNA in human glioblastoma cell line T98G using pSPORT1 plasmid (marketed by GIBCOBRL), as a vector, by Super Script Plasmid System for cDNA Synthesis and Plasmid Cloning (Trade name, marketed by GIBCOBRL). Next, after preparing 27 mer biotinylated primer OA004-F1;

5' biotin-ATGCACATCTTCAAGCATGCTCAG-3' (SEQ ID NO: 28) based on the information of the nucleotide sequence obtained by SST, plasmids hybridized specifically with the biotinylated primer were recovered from the cDNA library according to the method of Gene Trapper Kit and then transfected into *E. coli* DH10B. Colony hybridization with OA004 SST cDNA which was labeled with ³²P-dCTP, as a probe, was performed by using Random Primer DNA Labeling kit (Trade name, marketed by Takara Shuzo Co.) according to a known method to isolate a positive clone and to prepare a plasmid. After the nucleotide sequence at the 5'-side was determined to confirm that the nucleotide sequence of OA004 SST cDNA was present, a full-length nucleotide sequence was determined, cDNA sequences shown in SEQ ID NOs: 18 and 21 were obtained and named OA004-FG and OA004-LD, respectively. Furthermore, open reading frames were determined and deduced amino acid sequences, and sequences shown in SEQ ID NOs: 16 and 17 and NOs: 19 and 20, respectively, were obtained.

It was indicated from the results of homology search for the public database of the nucleic acid sequences by using BLASTN and FASTA, and for the public database of the amino acid sequences by using BLASTX, BLASTP and FASTA, that there was no sequence identical to the polypeptide sequences of OA004-FG and OA004-LD of the present invention and the nucleotide sequences encoding them. From these results, it was proved that polypeptides of the present invention were new secretary proteins. However, as a results of homology search, BLASTX, BLASTP and FASTA revealed a significant homology between clones OA004-FG and OA004-LD (region of 151st to 353rd amino acids in SEQ ID NOs: 16 and 19) and C, elegans 52.8 kD protein (Hypothetical 52.8 kD protein (Caenorhabdtis elegans), SwissProt Accession YJ95_CAEEL, region of 238th to 453rd amino acids). Moreover, they revealed a significant homology between clones OA004-FG and OA004-LD (region of 236th to 319th amino acids in SEQ ID NOs: 16 and 19) and human presenillin-2 (presenillin-2 (Homo sapiens), Genbank Accession A56993, region of 340th to 416th amino acids). Based on these homologies, clones OA004-FG and OA004-LD and presenillin family were expected to share at least some activity.

## Claims

1. A polypeptide in substantially purified form having the amino acid sequence of residues 1 to 324 of SEQ ID NO: 7.

2. A cDNA encoding the polypeptide according to claim 1.

3. A replication or expression vector carrying the cDNA according to claim 2.

4. A host cell transformed with the replication or expression vector according to claim 3.

5. A method for producing the polypeptide according to claim 1 which comprising culturing the host cell according to claim 4 under conditions effective to express the polypeptide according to claim 1.

6. A monoclonal or polyclonal antibody against the polypeptide according to claim 1.


Sequence Listing

<110> Ono Pharmaceutical Co., Ltd.

<120> Novel polypeptides, cDNA coding these polypeptides and Use thereof

<130> ONF-2852PCT

<150> JP 9-358811
<151> 1997-12-26

<160> 28

<170> PatentIn Ver. 2.0


<210> 1
<211> 777
<212> PRT
<213> Homo sapiens

<400> 1
Met Asn Ala Asn Lys Asp Glu Arg Leu Lys Ala Arg Ser Gln Asp Phe
-36 -35                 -30                 -25

His Leu Phe Pro Ala Leu Met Met Leu Ser Met Thr Met Leu Phe Leu
-20                 -15                 -10                  -5

Pro Val Thr Gly Thr Leu Lys Gln Asn Ile Pro Arg Leu Lys Leu Thr
                  1               5                  10

Tyr Lys Asp Leu Leu Leu Ser Asn Ser Cys Ile Pro Phe Leu Gly Ser
        15                  20                  25

Ser Glu Gly Leu Asp Phe Gln Thr Leu Leu Leu Asp Glu Glu Arg Gly
    30                  35                  40

Arg Leu Leu Leu Gly Ala Lys Asp His Ile Phe Leu Leu Ser Leu Val
 45                  50                  55                  60

Asp Leu Asn Lys Asn Phe Lys Lys Ile Tyr Trp Pro Ala Ala Lys Glu
                 65                  70                  75

Arg Val Glu Leu Cys Lys Leu Ala Gly Lys Asp Ala Asn Thr Glu Cys
             80                  85                  90

Ala Asn Phe Ile Arg Val Leu Gln Pro Tyr Asn Lys Thr His Ile Tyr
        95                 100                 105

Val Cys Gly Thr Gly Ala Phe His Pro Ile Cys Gly Tyr Ile Asp Leu
    110                 115                 120

Gly Val Tyr Lys Glu Asp Ile Ile Phe Lys Leu Asp Thr Arg Asn Leu
125                 130                 135                 140

Glu Ser Gly Arg Leu Lys Cys Pro Phe Asp Pro Gln Gln Pro Phe Ala
                145                 150                 155

Ser Val Met Thr Asp Glu Tyr Leu Tyr Ser Gly Thr Ala Ser Asp Phe
            160                 165                 170

Leu Gly Lys Asp Thr Ala Phe Thr Arg Ser Leu Gly Pro Thr His Asp
        175                 180                 185

His His Tyr Ile Arg Thr Asp Ile Ser Glu His Tyr Trp Leu Asn Gly
    190                 195                 200

Ala Lys Phe Ile Gly Thr Phe Phe Ile Pro Asp Thr Tyr Asn Pro Asp
205                 210                 215                 220

Asp Asp Lys Ile Tyr Phe Phe Phe Arg Glu Ser Ser Gln Glu Gly Ser
                225                 230                 235

Thr Ser Asp Lys Thr Ile Leu Ser Arg Val Gly Arg Val Cys Lys Asn
            240                 245                 250

Asp Val Gly Gly Gln Arg Ser Leu Ile Asn Lys Trp Thr Thr Phe Leu
        255                 260                 265

Lys Ala Arg Leu Ile Cys Ser Ile Pro Gly Ser Asp Gly Ala Asp Thr
    270                 275                 280

Tyr Phe Asp Glu Leu Gln Asp Ile Tyr Leu Leu Pro Thr Arg Asp Glu
285                 290                 295                 300

Arg Asn Pro Val Val Tyr Gly Val Phe Thr Thr Thr Ser Ser Ile Phe
                305                 310                 315

Lys Gly Ser Ala Val Cys Val Tyr Ser Met Ala Asp Ile Arg Ala Val
            320                 325                 330

Phe Asn Gly Pro Tyr Ala His Lys Glu Ser Ala Asp His Arg Trp Val
        335                 340                 345

Gln Tyr Asp Gly Arg Ile Pro Tyr Pro Arg Pro Gly Thr Cys Pro Ser
    350                 355                 360

Lys Thr Tyr Asp Pro Leu Ile Lys Ser Thr Arg Asp Phe Pro Asp Asp
365                 370                 375                 380

Val Ile Ser Phe Ile Lys Arg His Ser Val Met Tyr Lys Ser Val Tyr
                385                 390                 395

Pro Val Ala Gly Gly Pro Thr Phe Lys Arg Ile Asn Val Asp Tyr Arg
            400                 405                 410

Leu Thr Gln Ile Val Val Asp His Val Ile Ala Glu Asp Gly Gln Tyr
        415                 420                 425

Asp Val Met Phe Leu Gly Thr Asp Ile Gly Thr Val Leu Lys Val Val
    430                 435                 440

Ser Ile Ser Lys Glu Lys Trp Asn Met Glu Glu Val Val Leu Glu Glu
445                 450                 455                 460

Leu Gln Ile Phe Lys His Ser Ser Ile Ile Leu Asn Met Glu Leu Ser
                465                 470                 475

Leu Lys Gln Gln Gln Leu Tyr Ile Gly Ser Arg Asp Gly Leu Val Gln
            480                 485                 490

Leu Ser Leu His Arg Cys Asp Thr Tyr Gly Lys Ala Cys Ala Asp Cys
        495                 500                 505

Cys Leu Ala Arg Asp Pro Tyr Cys Ala Trp Asp Gly Asn Ala Cys Ser
    510                 515                 520

Arg Tyr Ala Pro Thr Ser Lys Arg Arg Ala Arg Arg Gln Asp Val Lys
525                 530                 535                 540

Tyr Gly Asp Pro Ile Thr Gln Cys Trp Asp Ile Glu Asp Ser Ile Ser
                545                 550                 555

His Glu Thr Ala Asp Glu Lys Val Ile Phe Gly Ile Glu Phe Asn Ser
            560                 565                 570

Thr Phe Leu Glu Cys Ile Pro Lys Ser Gln Gln Ala Thr Ile Lys Trp
        575                 580                 585

Tyr Ile Gln Arg Ser Gly Asp Glu His Arg Glu Glu Leu Lys Pro Asp
    590                 595                 600

Glu Arg Ile Ile Lys Thr Glu Tyr Gly Leu Leu Ile Arg Ser Leu Gln
605                 610                 615                 620

Lys Lys Asp Ser Gly Met Tyr Tyr Cys Lys Ala Gln Glu His Thr Phe
                625                 630                 635

Ile His Thr Ile Val Lys Leu Thr Leu Asn Val Ile Glu Asn Glu Gln
            640                 645                 650

Met Glu Asn Thr Gln Arg Ala Glu His Glu Glu Gly Gln Val Lys Asp
        655                 660                 665

Leu Leu Ala Glu Ser Arg Leu Arg Tyr Lys Asp Tyr Ile Gln Ile Leu
    670                 675                 680

Ser Ser Pro Asn Phe Ser Leu Asp Gln Tyr Cys Glu Gln Met Trp His
685                 690                 695                 700

Arg Glu Lys Arg Arg Gln Arg Asn Lys Gly Gly Pro Lys Trp Lys His
                705                 710                 715

Met Gln Glu Met Lys Lys Lys Arg Asn Arg Arg His His Arg Asp Leu
            720                 725                 730

Asp Glu Leu Pro Arg Ala Val Ala Thr
        735                 740

<210> 2
<211> 2331
<212> DNA
<213> Homo sapiens

<400> 2
atgaatgcta ataaagatga aagacttaaa gccagaagcc aagattttca cctttttcct      60

gctttgatga tgctaagcat gaccatgttg tttcttccag tcactggcac tttgaagcaa     120

aatattccaa gactcaagct aacctacaaa gacttgctgc tttcaaatag ctgtattccc     180

tttttgggtt catcagaagg actggatttt caaactcttc tcttagatga ggaaagaggc     240

aggctgctct tgggagccaa agaccacatc tttctactca gtctggttga cttaaacaaa     300

aattttaaga agatttattg gcctgctgca aaggaacggg tggaattatg taaattagct     360

gggaaagatg ccaatacaga atgtgcaaat ttcatcagag tacttcagcc ctataacaaa     420

actcacatat atgtgtgtgg aactggagca tttcatccaa tatgtgggta tattgatctt 480
ggagtctaca aggaggatat tatattcaaa ctagacacac gtaatttgga gtctggcaga 540
ctgaaatgtc ctttcgatcc tcagcagcct tttgcttcag taatgacaga tgagtacctc 600
tactctggaa cagcttctga tttccttggc aaagatactg cattcactcg atcccttggg 660
cctactcatg accaccacta catcagaact gacatttcag agcactactg gctcaatgga 720
gcaaaactta ttggaacttt cttcatacca gacacctaca atccagatga tgataaaata 780
tatttcttct ttcgtgaatc atctcaagaa ggcagtacct ccgataaaac catcctttct 840
cgagttggaa gagtttgtaa gaatgatgta ggaggacaac gcagcctgat aaacaagtgg 900
acgacttttc ttaaggccag actgatttgc tcaattcctg gaagtgatgg ggcagatact 960
tactttgatg agcttcaaga tatttattta ctccccacaa gagatgaaag aaatcctgta 1020
gtatatggag tctttactac aaccagctcc atcttcaaag gctctgctgt ttgtgtgtat 1080
agcatggctg acatcagagc agtttttaat ggtccatatg ctcataagga aagtgcagac 1140
catcgttggg tgcagtatga tgggagaatt ccttatccac ggcctggtac atgtccaagc 1200
aaaacctatg acccactgat taagtccacc cgagattttc cagatgatgt catcagtttc 1260
ataaagcggc actctgtgat gtataagtcc gtatacccag ttgcaggagg accaacgttc 1320
aagagaatca atgtggatta cagactgaca cagatagtgg tggatcatgt cattgcagaa 1380
gatggccagt acgatgtaat gtttcttgga acagacattg gaactgtcct caaagttgtc 1440
agcatttcaa aggaaaagtg gaatatggaa gaggtagtgc tggaggagtt gcagatattc 1500
aagcactcat caatcatctt gaacatggaa ttgtctctga agcagcaaca attgtacatt 1560
ggttcccgag atggattagt tcagctctcc ttgcacagat gcgacactta tgggaaagct 1620
tgcgcagact gttgtcttgc cagagacccc tactgtgcct gggatggaaa tgcatgctct 1680
cgatatgctc ctacttctaa aaggagagct agacgccaag atgtaaaata tggcgaccca 1740
atcacccagt gctgggacat cgaagacagc attagtcatg aaactgctga tgaaaaggtg 1800
atttttggca ttgaatttaa ctcaaccttt ctggaatgta tacctaaatc ccaacaagca 1860
actattaaat ggtatatcca gaggtcaggg gatgagcatc gagaggagtt gaagcccgat 1920
gaaagaatca tcaaaacgga atatgggcta ctgattcgaa gtttgcagaa gaaggattct 1980
gggatgtatt actgcaaagc ccaggagcac actttcatcc acaccatagt gaagctgact 2040
ttgaatgtca ttgagaatga acagatggaa aatacccaga gggcagagca tgaggagggg 2100
caggtcaagg atctattggc tgagtcacgg ttgagataca aagactacat ccaaatcctt 2160
agcagcccaa acttcagcct cgaccagtac tgcgaacaga tgtggcacag ggagaagcgg 2220
agacagagaa acaagggggg cccaaagtgg aagcacatgc aggaaatgaa gaagaaacga 2280

aatcgaagac atcacagaga cctggatgag ctccctagag ctgtagccac g          2331

<210> 3
<211> 3880
<212> DNA
<213> Homo sapiens

<220>
<223> Origin; human brain - derived  clone OM007

<220>
<221> CDS
<222> (88)..(2418)

<220>
<221> sig peptide
<222> (88)..(195)

<220>
<221> mat peptide
<222> (196)..(2418)

<400> 3
caccttacca actgcagatc ttgggactca tcagcctcaa taattatatt aaattaacac      60

catttgaaag agaacattgt tttcatc atg aat gct aat aaa gat gaa aga      111
                              Met Asn Ala Asn Lys Asp Glu Arg
                              -36 -35                 -30

ctt aaa gcc aga agc caa gat ttt cac ctt ttt cct gct ttg atg atg      159
Leu Lys Ala Arg Ser Gln Asp Phe His Leu Phe Pro Ala Leu Met Met
            -25                 -20                 -15

cta agc atg acc atg ttg ttt ctt cca gtc act ggc act ttg aag caa      207
Leu Ser Met Thr Met Leu Phe Leu Pro Val Thr Gly Thr Leu Lys Gln
        -10                 -5                  1

aat att cca aga ctc aag cta acc tac aaa gac ttg ctg ctt tca aat      255
Asn Ile Pro Arg Leu Lys Leu Thr Tyr Lys Asp Leu Leu Leu Ser Asn
  5                  10                  15                  20

agc tgt att ccc ttt ttg ggt tca tca gaa gga ctg gat ttt caa act      303
Ser Cys Ile Pro Phe Leu Gly Ser Ser Glu Gly Leu Asp Phe Gln Thr
                 25                  30                  35

ctt ctc tta gat gag gaa aga ggc agg ctg ctc ttg gga gcc aaa gac      351
Leu Leu Leu Asp Glu Glu Arg Gly Arg Leu Leu Leu Gly Ala Lys Asp
             40                  45                  50

cac atc ttt cta ctc agt ctg gtt gac tta aac aaa aat ttt aag aag      399
His Ile Phe Leu Leu Ser Leu Val Asp Leu Asn Lys Asn Phe Lys Lys
         55                  60                  65

att tat tgg cct gct gca aag gaa cgg gtg gaa tta tgt aaa tta gct      447
Ile Tyr Trp Pro Ala Ala Lys Glu Arg Val Glu Leu Cys Lys Leu Ala
     70                  75                  80

ggg aaa gat gcc aat aca gaa tgt gca aat ttc atc aga gta ctt cag      495
Gly Lys Asp Ala Asn Thr Glu Cys Ala Asn Phe Ile Arg Val Leu Gln
 85                  90                  95                 100

ccc tat aac aaa act cac ata tat gtg tgt gga act gga gca ttt cat      543

Pro Tyr Asn Lys Thr His Ile Tyr Val Cys Gly Thr Gly Ala Phe His
                105                 110                 115

cca ata tgt ggg tat att gat ctt gga gtc tac aag gag gat att ata      591
Pro Ile Cys Gly Tyr Ile Asp Leu Gly Val Tyr Lys Glu Asp Ile Ile
            120                 125                 130

ttc aaa cta gac aca cgt aat ttg gag tct ggc aga ctg aaa tgt cct      639
Phe Lys Leu Asp Thr Arg Asn Leu Glu Ser Gly Arg Leu Lys Cys Pro
        135                 140                 145

ttc gat cct cag cag cct ttt gct tca gta atg aca gat gag tac ctc      687
Phe Asp Pro Gln Gln Pro Phe Ala Ser Val Met Thr Asp Glu Tyr Leu
    150                 155                 160

tac tct gga aca gct tct gat ttc ctt ggc aaa gat act gca ttc act      735
Tyr Ser Gly Thr Ala Ser Asp Phe Leu Gly Lys Asp Thr Ala Phe Thr
165                 170                 175                 180

cga tcc ctt ggg cct act cat gac cac cac tac atc aga act gac att      783
Arg Ser Leu Gly Pro Thr His Asp His His Tyr Ile Arg Thr Asp Ile
                185                 190                 195

tca gag cac tac tgg ctc aat gga gca aaa ttt att gga act ttc ttc      831
Ser Glu His Tyr Trp Leu Asn Gly Ala Lys Phe Ile Gly Thr Phe Phe
            200                 205                 210
ata cca gac acc tac aat cca gat gat gat aaa ata tat ttc ttc ttt      879
Ile Pro Asp Thr Tyr Asn Pro Asp Asp Asp Lys Ile Tyr Phe Phe Phe
        215                 220                 225

cgt gaa tca tct caa gaa ggc agt acc tcc gat aaa acc atc ctt tct      927
Arg Glu Ser Ser Gln Glu Gly Ser Thr Ser Asp Lys Thr Ile Leu Ser
    230                 235                 240

cga gtt gga aga gtt tgt aag aat gat gta gga gga caa cgc agc ctg      975
Arg Val Gly Arg Val Cys Lys Asn Asp Val Gly Gly Gln Arg Ser Leu
245                 250                 255                 260

ata aac aag tgg acg act ttt ctt aag gcc aga ctg att tgc tca att      1023
Ile Asn Lys Trp Thr Thr Phe Leu Lys Ala Arg Leu Ile Cys Ser Ile
                265                 270                 275

cct gga agt gat ggg gca gat act tac ttt gat gag ctt caa gat att      1071
Pro Gly Ser Asp Gly Ala Asp Thr Tyr Phe Asp Glu Leu Gln Asp Ile
            280                 285                 290

tat tta ctc ccc aca aga gat gaa aga aat cct gta gta tat gga gtc      1119
Tyr Leu Leu Pro Thr Arg Asp Glu Arg Asn Pro Val Val Tyr Gly Val
        295                 300                 305

ttt act aca acc agc tcc atc ttc aaa ggc tct gct gtt tgt gtg tat      1167
Phe Thr Thr Thr Ser Ser Ile Phe Lys Gly Ser Ala Val Cys Val Tyr
    310                 315                 320

agc atg gct gac atc aga gca gtt ttt aat ggt cca tat gct cat aag      1215
Ser Met Ala Asp Ile Arg Ala Val Phe Asn Gly Pro Tyr Ala His Lys
325                 330                 335                 340

gaa agt gca gac cat cgt tgg gtg cag tat gat ggg aga att cct tat      1263
Glu Ser Ala Asp His Arg Trp Val Gln Tyr Asp Gly Arg Ile Pro Tyr
                345                 350                 355

cca cgg cct ggt aca tgt cca agc aaa acc tat gac cca ctg att aag      1311

Pro Arg Pro Gly Thr Cys Pro Ser Lys Thr Tyr Asp Pro Leu Ile Lys
            360                 365                 370

tcc acc cga gat ttt cca gat gat gtc atc agt ttc ata aag cgg cac      1359
Ser Thr Arg Asp Phe Pro Asp Asp Val Ile Ser Phe Ile Lys Arg His
        375                 380                 385

tct gtg atg tat aag tcc gta tac cca gtt gca gga gga cca acg ttc      1407
Ser Val Met Tyr Lys Ser Val Tyr Pro Val Ala Gly Gly Pro Thr Phe
    390                 395                 400

aag aga atc aat gtg gat tac aga ctg aca cag ata gtg gtg gat cat      1455
Lys Arg Ile Asn Val Asp Tyr Arg Leu Thr Gln Ile Val Val Asp His
405                 410                 415                 420

gtc att gca gaa gat ggc cag tac gat gta atg ttt ctt gga aca gac      1503
Val Ile Ala Glu Asp Gly Gln Tyr Asp Val Met Phe Leu Gly Thr Asp
                425                 430                 435

att gga act gtc ctc aaa gtt gtc agc att tca aag gaa aag tgg aat      1551
Ile Gly Thr Val Leu Lys Val Val Ser Ile Ser Lys Glu Lys Trp Asn
            440                 445                 450

atg gaa gag gta gtg ctg gag gag ttg cag ata ttc aag cac tca tca      1599
Met Glu Glu Val Val Leu Glu Glu Leu Gln Ile Phe Lys His Ser Ser
        455                 460                 465

atc atc ttg aac atg gaa ttg tct ctg aag cag caa caa ttg tac att      1647
Ile Ile Leu Asn Met Glu Leu Ser Leu Lys Gln Gln Gln Leu Tyr Ile
    470                 475                 480

ggt tcc cga gat gga tta gtt cag ctc tcc ttg cac aga tgc gac act      1695
Gly Ser Arg Asp Gly Leu Val Gln Leu Ser Leu His Arg Cys Asp Thr
485                 490                 495                 500

tat ggg aaa gct tgc gca gac tgt tgt ctt gcc aga gac ccc tac tgt      1743
Tyr Gly Lys Ala Cys Ala Asp Cys Cys Leu Ala Arg Asp Pro Tyr Cys
                505                 510                 515

gcc tgg gat gga aat gca tgc tct cga tat gct cct act tct aaa agg      1791
Ala Trp Asp Gly Asn Ala Cys Ser Arg Tyr Ala Pro Thr Ser Lys Arg
            520                 525                 530

aga gct aga cgc caa gat gta aaa tat ggc gac cca atc acc cag tgc      1839
Arg Ala Arg Arg Gln Asp Val Lys Tyr Gly Asp Pro Ile Thr Gln Cys
        535                 540                 545

tgg gac atc gaa gac agc att agt cat gaa act gct gat gaa aag gtg      1887
Trp Asp Ile Glu Asp Ser Ile Ser His Glu Thr Ala Asp Glu Lys Val
    550                 555                 560

att ttt ggc att gaa ttt aac tca acc ttt ctg gaa tgt ata cct aaa      1935
Ile Phe Gly Ile Glu Phe Asn Ser Thr Phe Leu Glu Cys Ile Pro Lys
565                 570                 575                 580

tcc caa caa gca act att aaa tgg tat atc cag agg tca ggg gat gag      1983
Ser Gln Gln Ala Thr Ile Lys Trp Tyr Ile Gln Arg Ser Gly Asp Glu
                585                 590                 595

cat cga gag gag ttg aag ccc gat gaa aga atc atc aaa acg gaa tat      2031
His Arg Glu Glu Leu Lys Pro Asp Glu Arg Ile Ile Lys Thr Glu Tyr
            600                 605                 610

ggg cta ctg att cga agt ttg cag aag aag gat tct ggg atg tat tac     2079
Gly Leu Leu Ile Arg Ser Leu Gln Lys Lys Asp Ser Gly Met Tyr Tyr
        615                 620                 625

tgc aaa gcc cag gag cac act ttc atc cac acc ata gtg aag ctg act     2127
Cys Lys Ala Gln Glu His Thr Phe Ile His Thr Ile Val Lys Leu Thr
    630                 635                 640

ttg aat gtc att gag aat gaa cag atg gaa aat acc cag agg gca gag     2175
Leu Asn Val Ile Glu Asn Glu Gln Met Glu Asn Thr Gln Arg Ala Glu
645                 650                 655                 660

cat gag gag ggg cag gtc aag gat cta ttg gct gag tca cgg ttg aga     2223
His Glu Glu Gly Gln Val Lys Asp Leu Leu Ala Glu Ser Arg Leu Arg
                665                 670                 675

tac aaa gac tac atc caa atc ctt agc agc cca aac ttc agc ctc gac     2271
Tyr Lys Asp Tyr Ile Gln Ile Leu Ser Ser Pro Asn Phe Ser Leu Asp
            680                 685                 690

cag tac tgc gaa cag atg tgg cac agg gag aag cgg aga cag aga aac     2319
Gln Tyr Cys Glu Gln Met Trp His Arg Glu Lys Arg Arg Gln Arg Asn
        695                 700                 705

aag ggg ggc cca aag tgg aag cac atg cag gaa atg aag aag aaa cga     2367
Lys Gly Gly Pro Lys Trp Lys His Met Gln Glu Met Lys Lys Lys Arg
    710                 715                 720

aat cga aga cat cac aga gac ctg gat gag ctc cct aga gct gta gcc     2415
Asn Arg Arg His His Arg Asp Leu Asp Glu Leu Pro Arg Ala Val Ala
725                 730                 735                 740

acg tagtttctca cttaatttaa agaaaagaat tccttaccta taaaaacatt     2468
Thr

gccttctgtt ttgtatatcc cttatagtaa ttcataaatg cttcccatgg agttttgcta     2528

aggcacaaga caataatctg aataagacaa tatgtgatga atataagaaa gggcaaaaaa     2588

ttcatttgaa ccagttttcc aagaacaaat cttgcacaag caaagtataa gaattatcct     2648

aaaaataggg ggtttacagt tgtaaatgtt ttatgttttg agttttggaa tttattgtca     2708

tgtaaatagt tgagctaagc aagccccgaa tttgatagtg tataaggtgc tttattccct     2768

cgaatgtcca ttaagcatgg aatttaccat gcagttgtgc tatgttctta tgaacagata     2828

tatcattcct attgagaacc agctaccttg tggtagggaa taagaggtca gacacaaatt     2888

aagacaactc ccattatcaa caggaacttt ctcagtgagc cattcactcc tggagaatgg     2948

tataggaatt tggagaggtg cattatttct ttctggccac tggggttaaa tttagtgtac     3008

tacaacattg atttactgaa gggcactaat gtttccccca ggatttctat tgactagtca     3068

ggagtaacag gttcacagag agaagttggt gcttagttat gtgtttttta gagtatatac     3128

taagctctac agggacagaa tgcttaataa atactttaat aagatatggg aaaatatttt     3188

aataaaacaa ggaaaacata atgatgtata atgcatcctg atgggaaggc atgcagatgg     3248

gatttgttag aagacagaag gaaagacagc cataaattct ggctttgggg aaaactcata     3368

tccccatgaa aaggaagaac aatcacaaat aaagtgagag taatgtaatg gagctctttt 3368

cactagggta taagtagctg ccaatttgta attcatctgt taaaaaaaat ctagattata 3428

acaaactgct agcaaaatct gaggaaacat aaattcttct gaagaatcat aggaagagta 3488

gacattttat ttataaccaa tgatatttca gtatatattt tctctctttt aaaaaatatt 3548

tatcatactc tgtatattat ttctttttac tgcctttatt ctctcctgta tattggattt 3608

tgtgattata tttgagtgaa taggagaaaa caatatataa cacacagaga attaagaaaa 3668

tgacatttct ggggagtggg gatatatatt tgttgaataa cagaacgagt gtaaaatttt 3728

aacaacggaa agggttaaat taactctttg acatcttcac tcaacctttt ctcattgctg 3788

agttaatctg ttgtaattgt agtattgttt ttgtaattta acaataaata agcctgctac 3848

atgtaaaaag aaccaaaaaa aaaaaaaaaa aa 3880

<210> 4
<211> 356
<212> PRT
<213> Homo sapiens

<400> 4
Met His His Gln Trp Leu Leu Leu Ala Ala Cys Phe Trp Val Ile Phe
-17 -15 -10 -5

Met Phe Met Val Ala Ser Lys Phe Ile Thr Leu Thr Phe Lys Asp Pro
1               5                   10                  15

Asp Val Tyr Ser Ala Lys Gln Glu Phe Leu Phe Leu Thr Thr Met Pro
            20                  25                  30

Glu Val Arg Lys Leu Pro Glu Glu Lys His Ile Pro Glu Glu Leu Lys
        35                  40                  45

Pro Thr Gly Lys Glu Leu Pro Asp Ser Gln Leu Val Gln Pro Leu Val
    50                  55                  60

Tyr Met Glu Arg Leu Glu Leu Ile Arg Asn Val Cys Arg Asp Asp Ala
65 70 75

Leu Lys Asn Leu Ser His Thr Pro Val Ser Lys Phe Val Leu Asp Arg
80 85 90 95

Ile Phe Val Cys Asp Lys His Lys Ile Leu Phe Cys Gln Thr Pro Lys
            100                 105                 110

Val Gly Asn Thr Gln Trp Lys Lys Val Leu Ile Val Leu Asn Gly Ala
        115                 120                 125

Phe Ser Ser Ile Glu Glu Ile Pro Glu Asn Val Val His Asp His Glu
    130                 135                 140

Lys Asn Gly Leu Pro Arg Leu Ser Ser Phe Ser Asp Ala Glu Ile Gln
145 150 155

Lys Arg Leu Lys Thr Tyr Phe Lys Phe Phe Ile Val Arg Asp Pro Phe

160                 165                 170                 175

Glu Arg Leu Ile Ser Ala Phe Lys Asp Lys Phe Val His Asn Pro Arg
                180                 185                 190

Phe Glu Pro Trp Tyr Arg His Glu Ile Ala Pro Gly Ile Ile Arg Lys
            195                 200                 205

Tyr Arg Arg Asn Arg Thr Glu Thr Arg Gly Ile Gln Phe Glu Asp Phe
        210                 215                 220

Val Arg Tyr Leu Gly Asp Pro Asn His Arg Trp Leu Asp Leu Gln Phe
    225                 230                 235

Gly Asp His Ile Ile His Trp Val Thr Tyr Val Glu Leu Cys Ala Pro
240                 245                 250                 255

Cys Glu Ile Met Tyr Ser Val Ile Gly His His Glu Thr Leu Glu Asp
                260                 265                 270

Asp Ala Pro Tyr Ile Leu Lys Glu Ala Gly Ile Asp His Leu Val Ser
            275                 280                 285

Tyr Pro Thr Ile Pro Pro Gly Ile Thr Val Tyr Asn Arg Thr Lys Val
        290                 295                 300

Glu His Tyr Phe Leu Gly Ile Ser Lys Arg Asp Ile Arg Arg Leu Tyr
    305                 310                 315

Ala Arg Phe Glu Gly Asp Phe Lys Leu Phe Gly Tyr Gln Lys Pro Asp
320                 325                 330                 335

Phe Leu Leu Asn

<210> 5
<211> 1068
<212> DNA
<213> Homo sapiens

<400> 5
atgcaccacc agtggcttct gctggccgca tgcttttggg tgattttcat gttcatggtg      60

gctagcaagt tcatcacgtt gacctttaaa gacccagatg tgtacagtgc caaacaggag     120

tttctgttcc tgacaaccat gccggaagtg aggaagttgc cagaagagaa gcacattcct     180

gaggaactga agccaactgg gaaggagctt ccagacagcc agctcgttca gcccctggtc     240

tacatggagc gcctggaact catcagaaac gtctgcaggg atgatgccct gaagaatctc     300

tcgcacactc ctgtctccaa gtttgtcctg gaccgaatat ttgtctgtga caagcacaag     360

attcttttct gccagactcc caaagtgggc aacacccagt ggaagaaagt gctgattgtt     420

ctaaatggag cattttcttc cattgaggag atccccgaaa acgtggtgca cgaccacgag     480

aagaacggcc ttcctcggct ctcttccttc agtgatgcag aaattcagaa gcgattgaaa     540

acatacttca agttttttat tgtaagagat cccttcgaaa gacttatttc tgcatttaag     600

gataaatttg ttcacaatcc ccggtttgag ccttggtaca ggcatgagat tgctcctggc     660

atcatcagaa aatacaggag gaaccggaca gagacccggg ggatccagtt tgaagatttc     720

gtgcgctacc tcggcgatcc gaaccacaga tggctagacc ttcagtttgg ggaccacatc     780

attcactggg tgacgtatgt agagctctgt gctccctgtg agataatgta cagtgtgatt     840

ggacaccacg agaccctgga ggacgatgcc ccatacatct taaaagaggc tggcattgac     900

cacctggtgt catacccgac tatccctccg ggcattaccg tgtataacag aaccaaggtg     960

gagcactatt tcctgggcat cagcaaacga gacatccgac gcctgtatgc ccgtttcgaa    1020

ggggacttta agctctttgg gtaccagaaa ccagactttt tgctaaac                 1068

<210> 6
<211> 2479
<212> DNA
<213> Homo sapiens

<220>
<223> Origin; human brain - derived  clone OMB096

<220>
<221> CDS
<222> (6)..(1073)

<220>
<221> sig peptide
<222> (6)..(56)

<220>
<221> mat peptide
<222> (57)..(1073)

<400> 6
acaac atg cac cac cag tgg ctt ctg ctg gcc gca tgc ttt tgg gtg        47
      Met His His Gln Trp Leu Leu Leu Ala Ala Cys Phe Trp Val
      -17     -15                 -10                 -5

att ttc atg ttc atg gtg gct agc aag ttc atc acg ttg acc ttt aaa      95
Ile Phe Met Phe Met Val Ala Ser Lys Phe Ile Thr Leu Thr Phe Lys
            1               5                   10

gac cca gat gtg tac agt gcc aaa cag gag ttt ctg ttc ctg aca acc     143
Asp Pro Asp Val Tyr Ser Ala Lys Gln Glu Phe Leu Phe Leu Thr Thr
    15                  20                  25

atg ccg gaa gtg agg aag ttg cca gaa gag aag cac att cct gag gaa     191
Met Pro Glu Val Arg Lys Leu Pro Glu Glu Lys His Ile Pro Glu Glu
30                  35                  40                  45

ctg aag cca act ggg aag gag ctt cca gac agc cag ctc gtt cag ccc     239
Leu Lys Pro Thr Gly Lys Glu Leu Pro Asp Ser Gln Leu Val Gln Pro
                50                  55                  60

ctg gtc tac atg gag cgc ctg gaa ctc atc aga aac gtc tgc agg gat     287
Leu Val Tyr Met Glu Arg Leu Glu Leu Ile Arg Asn Val Cys Arg Asp
            65                  70                  75

gat gcc ctg aag aat ctc tcg cac act cct gtc tcc aag ttt gtc ctg     335

Asp Ala Leu Lys Asn Leu Ser His Thr Pro Val Ser Lys Phe Val Leu
        80                  85                  90

gac cga ata ttt gtc tgt gac aag cac aag att ctt ttc tgc cag act      383
Asp Arg Ile Phe Val Cys Asp Lys His Lys Ile Leu Phe Cys Gln Thr
    95                  100                 105

ccc aaa gtg ggc aac acc cag tgg aag aaa gtg ctg att gtt cta aat      431
Pro Lys Val Gly Asn Thr Gln Trp Lys Lys Val Leu Ile Val Leu Asn
110                 115                 120                 125

gga gca ttt tct tcc att gag gag atc ccc gaa aac gtg gtg cac gac      479
Gly Ala Phe Ser Ser Ile Glu Glu Ile Pro Glu Asn Val Val His Asp
                130                 135                 140

cac gag aag aac ggc ctt cct cgg ctc tct tcc ttc agt gat gca gaa      527
His Glu Lys Asn Gly Leu Pro Arg Leu Ser Ser Phe Ser Asp Ala Glu
            145                 150                 155

att cag aag cga ttg aaa aca tac ttc aag ttt ttt att gta aga gat      575
Ile Gln Lys Arg Leu Lys Thr Tyr Phe Lys Phe Phe Ile Val Arg Asp
        160                 165                 170

ccc ttc gaa aga ctt att tct gca ttt aag gat aaa ttt gtt cac aat      623
Pro Phe Glu Arg Leu Ile Ser Ala Phe Lys Asp Lys Phe Val His Asn
    175                 180                 185

ccc cgg ttt gag cct tgg tac agg cat gag att gct cct ggc atc atc      671
Pro Arg Phe Glu Pro Trp Tyr Arg His Glu Ile Ala Pro Gly Ile Ile
190                 195                 200                 205

aga aaa tac agg agg aac cgg aca gag acc cgg ggg atc cag ttt gaa      719
Arg Lys Tyr Arg Arg Asn Arg Thr Glu Thr Arg Gly Ile Gln Phe Glu
                210                 215                 220

gat ttc gtg cgc tac ctc ggc gat ccg aac cac aga tgg cta gac ctt      767
Asp Phe Val Arg Tyr Leu Gly Asp Pro Asn His Arg Trp Leu Asp Leu
            225                 230                 235

cag ttt ggg gac cac atc att cac tgg gtg acg tat gta gag ctc tgt      815
Gln Phe Gly Asp His Ile Ile His Trp Val Thr Tyr Val Glu Leu Cys
        240                 245                 250

gct ccc tgt gag ata atg tac agt gtg att gga cac cac gag acc ctg      863
Ala Pro Cys Glu Ile Met Tyr Ser Val Ile Gly His His Glu Thr Leu
    255                 260                 265

gag gac gat gcc cca tac atc tta aaa gag gct ggc att gac cac ctg      911
Glu Asp Asp Ala Pro Tyr Ile Leu Lys Glu Ala Gly Ile Asp His Leu
270                 275                 280                 285

gtg tca tac ccg act atc cct ccg ggc att acc gtg tat aac aga acc      959
Val Ser Tyr Pro Thr Ile Pro Pro Gly Ile Thr Val Tyr Asn Arg Thr
                290                 295                 300

aag gtg gag cac tat ttc ctg ggc atc agc aaa cga gac atc cga cgc     1007
Lys Val Glu His Tyr Phe Leu Gly Ile Ser Lys Arg Asp Ile Arg Arg
            305                 310                 315

ctg tat gcc cgt ttc gaa ggg gac ttt aag ctc ttt ggg tac cag aaa     1055
Leu Tyr Ala Arg Phe Glu Gly Asp Phe Lys Leu Phe Gly Tyr Gln Lys
        320                 325                 330

cca gac ttt ttg cta aac taatgcataa gacctatgaa ttcaaatatc 1103
Pro Asp Phe Leu Leu Asn
335

tttattagac ctggggctaa ccaggtgaag atctgagccc agaaatgacc cttcctccac 1163

cacacccctc ctttgaggac gcccggggtc tcccacaggc ctgtgagttg cctcggcata 1223

tgacgcagaa ccccaactgt tacaacttag tttggatgta agatgctctg aggaccctgc 1283

ccacacccct gcgtgcatta ggatgtcgct ggcctttgct cacctcagag gggagaaaag 1343

gctaaagatt tgcagtttga cagcccagca gggaggaagc atcacacagc gttaggagcc 1403

gtttccttca ggtgttaagg aaggggatgc ccctgaggtt ctcctggcta gtcagggtgg 1463

cttcacccat cactggtggg ttgcaggaac agcacccagg actctgagga gggacagaga 1523

agcaaggggg ctgctgaaat cgcagagact tttgcagcat cagatctgag gagtaaaacg 1583

gcacctctgg ccttcatctt ggtgctgcga caattgtgga ggcaaagcat tctttctgtg 1643

actattttgt tcctgtagac agtcagcgat ggccagaggg tggtgtggtg tccaggggtc 1703

catctttcca gaatccatgc ctgtgtaatg ctggtccatg cttctgaacc tgtgtctgcc 1763

aagcgcctat ttcattcagc acaagacata cgattttaga aggtgagggg aggggaggct 1823

ttttctacct gagaagggga gtgtctttga gggccttaaa aggaccatgg cccaggaatg 1883

ggggcgctgg ttgggcttgg agctcaggct gctgtggatc ccggcgcatc agttctgact 1943

tgccttacct gggtggacag cagtgaatct ccacctgtct tctccaggga gctcccatgt 2003

tggggctgaa gacgagcagg ggcaacctgc cagcatcaca gaattcagtg tagtttatac 2063

atttcgattc ctttcatctc agcaaaatgg gcactgccag agccatttct gatcacacca 2123

ccatcctgga ccatgtgact ggaaggtggg taaccaagtt caccagcaat aaaacccagc 2183

gcccaggtag cctccagcag tgcggcttcc tggcaacaag gtaggccctg gtgcagggca 2243

agccgcagcg accatttcag ataccgtcca cagccaggac cgctgagaac tgggacagtt 2303

tcctgggatg agtgccagcc tgagcctgca tggtgccgcc gagcccgggg tggaggaggg 2363

agccaggctt cgcttcaagg cggcctctac cttttctcag aatggtttcc tgattgtgtc 2423

aatgtgaaag ttaaataaaa tttatgtgcc aaacctgaaa aaaaaaaaaa aaaaaa 2479


<210> 7
<211> 343
<212> PRT
<213> Homo sapiens

<400> 7
Met Trp Ser His Leu Asn Arg Leu Leu Phe Trp Ser Ile Phe Ser Ser
-19 -15 -10 -5

Val Thr Cys Arg Lys Ala Val Leu Asp Cys Glu Ala Met Lys Thr Asn

1 5 10

Glu Phe Pro Ser Pro Cys Leu Asp Ser Lys Thr Lys Val Val Met Lys
15 20 25

Gly Gln Asn Val Ser Met Phe Cys Ser His Lys Asn Lys Ser Leu Gln
30 35 40 45

Ile Thr Tyr Ser Leu Phe Arg Arg Lys Thr His Leu Gly Thr Gln Asp
    50                  55                  60

Gly Lys Gly Glu Pro Ala Ile Phe Asn Leu Ser Ile Thr Glu Ala His
65 70 75

Glu Ser Gly Pro Tyr Lys Cys Lys Ala Gln Val Thr Ser Cys Ser Lys
80 85 90

Tyr Ser Arg Asp Phe Ser Phe Thr Ile Val Asp Pro Val Thr Ser Pro
95 100 105

Val Leu Asn Ile Met Val Ile Gln Thr Glu Thr Asp Arg His Ile Thr
110 115 120 125

Leu His Cys Leu Ser Val Asn Gly Ser Leu Pro Ile Asn Tyr Thr Phe
    130                 135                 140

Phe Glu Asn His Val Ala Ile Ser Pro Ala Ile Ser Lys Tyr Asp Arg
145 150 155

Glu Pro Ala Glu Phe Asn Leu Thr Lys Lys Asn Pro Gly Glu Glu Glu
160 165 170

Glu Tyr Arg Cys Glu Ala Lys Asn Arg Leu Pro Asn Tyr Ala Thr Tyr
175 180 185

Ser His Pro Val Thr Met Pro Ser Thr Gly Gly Asp Ser Cys Pro Phe
190 195 200 205

Cys Leu Lys Leu Leu Leu Pro Gly Leu Leu Leu Leu Leu Val Val Ile
    210                 215                 220

Ile Leu Ile Leu Ala Phe Trp Val Leu Pro Lys Tyr Lys Thr Arg Lys
225 230 235

Ala Met Arg Asn Asn Val Pro Arg Asp Arg Gly Asp Thr Ala Met Glu
240 245 250

Val Gly Ile Tyr Ala Asn Ile Leu Glu Lys Gln Ala Lys Glu Glu Ser
255 260 265

Val Pro Glu Val Gly Ser Arg Pro Cys Val Ser Thr Ala Gln Asp Glu
270 275 280 285

Ala Lys His Ser Gln Glu Leu Gln Tyr Ala Thr Pro Val Phe Gln Glu
    290                 295                 300

Val Ala Pro Arg Glu Gln Glu Ala Cys Asp Ser Tyr Lys Ser Gly Tyr
305 310 315

Val Tyr Ser Glu Leu Asn Phe
320

<210> 8
<211> 1029
<212> DNA
<213> Homo sapiens

<400> 8
atgtggagcc atttgaacag gctcctcttc tggagcatat tttcttctgt cacttgtaga      60

aaagctgtat tggattgtga ggcaatgaaa acaaatgaat tcccttctcc atgtttggac     120

tcaaagacta aggtggttat gaagggtcaa aatgtatcta tgttttgttc ccataagaac     180

aaatcactgc agatcaccta ttcattgttt cgacgtaaga cacacctggg aacccaggat     240

ggaaaaggtg aacctgcgat ttttaaccta agcatcacag aagcccatga atcaggcccc     300

tacaaatgca aagcccaagt taccagctgt tcaaaataca gtcgtgactt cagcttcacg     360

attgtcgacc cggtgacttc cccagtgctg aacattatgg tcattcaaac agaaacagac     420

cgacatataa cattacattg cctctcagtc aatggctcgc tgcccatcaa ttacactttc     480

tttgaaaacc atgttgccat atcaccagct atttccaagt atgacaggga gcctgctgaa     540

tttaacttaa ccaagaagaa tcctggagaa gaggaagagt ataggtgtga agctaaaaac     600

agattgccta actatgcaac atacagtcac cctgtcacca tgccctcaac aggcggagac     660

agctgtcctt tctgtctgaa gctactactt ccagggttat tactgttgct ggtggtgata     720

atcctaattc tggctttttg ggtactgccc aaatacaaaa caagaaaagc tatgagaaat     780

aatgtgccca gggaccgtgg agacacagcc atggaagttg gaatctatgc aaatatcctt     840

gaaaaacaag caaaggagga atctgtgcca gaagtgggat ccaggccgtg tgtttccaca     900

gcccaagatg aggccaaaca ctcccaggag ctacagtatg ccacccccgt gttccaggag     960

gtggcaccaa gagagcaaga agcctgtgat tcttataaat ctggatatgt ctattctgaa    1020

ctcaacttc                                                            1029


<210> 9
<211> 1370
<212> DNA
<213> Homo sapiens

<220>
<223> Origin; human bone marrow stroma cell HAS 303 - derived  clone
OAF038-Leu

<220>
<221> CDS
<222> (7)..(1035)

<220>
<221> sig peptide
<222> (7)..(63)

<220>

<221> mat peptide
<222> (64)..(1035)

<400> 9
gggaga atg tgg agc cat ttg aac agg ctc ctc ttc tgg agc ata ttt        48
       Met Trp Ser His Leu Asn Arg Leu Leu Phe Trp Ser Ile Phe
       -19             -15                 -10

tct tct gtc act tgt aga aaa gct gta ttg gat tgt gag gca atg aaa        96
Ser Ser Val Thr Cys Arg Lys Ala Val Leu Asp Cys Glu Ala Met Lys
 -5                  1               5                  10

aca aat gaa ttc cct tct cca tgt ttg gac tca aag act aag gtg gtt       144
Thr Asn Glu Phe Pro Ser Pro Cys Leu Asp Ser Lys Thr Lys Val Val
            15                  20                  25

atg aag ggt caa aat gta tct atg ttt tgt tcc cat aag aac aaa tca       192
Met Lys Gly Gln Asn Val Ser Met Phe Cys Ser His Lys Asn Lys Ser
        30                  35                  40

ctg cag atc acc tat tca ttg ttt cga cgt aag aca cac ctg gga acc       240
Leu Gln Ile Thr Tyr Ser Leu Phe Arg Arg Lys Thr His Leu Gly Thr
    45                  50                  55

cag gat gga aaa ggt gaa cct gcg att ttt aac cta agc atc aca gaa       288
Gln Asp Gly Lys Gly Glu Pro Ala Ile Phe Asn Leu Ser Ile Thr Glu
60                  65                  70                  75

gcc cat gaa tca ggc ccc tac aaa tgc aaa gcc caa gtt acc agc tgt       336
Ala His Glu Ser Gly Pro Tyr Lys Cys Lys Ala Gln Val Thr Ser Cys
                80                  85                  90

tca aaa tac agt cgt gac ttc agc ttc acg att gtc gac ccg gtg act       384
Ser Lys Tyr Ser Arg Asp Phe Ser Phe Thr Ile Val Asp Pro Val Thr
            95                  100                 105

tcc cca gtg ctg aac att atg gtc att caa aca gaa aca gac cga cat       432
Ser Pro Val Leu Asn Ile Met Val Ile Gln Thr Glu Thr Asp Arg His
        110                 115                 120

ata aca tta cat tgc ctc tca gtc aat ggc tcg ctg ccc atc aat tac       480
Ile Thr Leu His Cys Leu Ser Val Asn Gly Ser Leu Pro Ile Asn Tyr
    125                 130                 135

act ttc ttt gaa aac cat gtt gcc ata tca cca gct att tcc aag tat       528
Thr Phe Phe Glu Asn His Val Ala Ile Ser Pro Ala Ile Ser Lys Tyr
140                 145                 150                 155

gac agg gag cct gct gaa ttt aac tta acc aag aag aat cct gga gaa       576
Asp Arg Glu Pro Ala Glu Phe Asn Leu Thr Lys Lys Asn Pro Gly Glu
                160                 165                 170

gag gaa gag tat agg tgt gaa gct aaa aac aga ttg cct aac tat gca       624
Glu Glu Glu Tyr Arg Cys Glu Ala Lys Asn Arg Leu Pro Asn Tyr Ala
            175                 180                 185

aca tac agt cac cct gtc acc atg ccc tca aca ggc gga gac agc tgt       672
Thr Tyr Ser His Pro Val Thr Met Pro Ser Thr Gly Gly Asp Ser Cys
        190                 195                 200

cct ttc tgt ctg aag cta cta ctt cca ggg tta tta ctg ttg ctg gtg       720
Pro Phe Cys Leu Lys Leu Leu Leu Pro Gly Leu Leu Leu Leu Leu Val
    205                 210                 215

gtg ata atc cta att ctg gct ttt tgg gta ctg ccc aaa tac aaa aca 768
Val Ile Ile Leu Ile Leu Ala Phe Trp Val Leu Pro Lys Tyr Lys Thr
220 225 230 235

aga aaa gct atg aga aat aat gtg ccc agg gac cgt gga gac aca gcc 816
Arg Lys Ala Met Arg Asn Asn Val Pro Arg Asp Arg Gly Asp Thr Ala
240 245 250

atg gaa gtt gga atc tat gca aat atc ctt gaa aaa caa gca aag gag 864
Met Glu Val Gly Ile Tyr Ala Asn Ile Leu Glu Lys Gln Ala Lys Glu
255 260 265

gaa tct gtg cca gaa gtg gga tcc agg ccg tgt gtt tcc aca gcc caa 912
Glu Ser Val Pro Glu Val Gly Ser Arg Pro Cys Val Ser Thr Ala Gln
270 275 280

gat gag gcc aaa cac tcc cag gag cta cag tat gcc acc ccc gtg ttc 960
Asp Glu Ala Lys His Ser Gln Glu Leu Gln Tyr Ala Thr Pro Val Phe
285 290 295

cag gag gtg gca cca aga gag caa gaa gcc tgt gat tct tat aaa tct 1008
Gln Glu Val Ala Pro Arg Glu Gln Glu Ala Cys Asp Ser Tyr Lys Ser
300 305 310 315

gga tat gtc tat tct gaa ctc aac ttc tgaaatttac agaaacaaac 1055
Gly Tyr Val Tyr Ser Glu Leu Asn Phe
320

tacatctcag ggtaaggatg ctttttatga agctgatttc catgaacaaa aagcaaactt 1115

gaggctgagg cgggtggatc acagggtcag gagatcaaga ccatcctggc taacacgatg 1175

aaaccccgtc tctactaaaa aatacaaaaa ttagccaggt gtggtggtgt gtgtgtgtag 1235

tcccagctac tcgggaggct gaggcaggag aatcgcttga gcccgggagg cagaggttgc 1295

agtgagccaa gatcgtgcca ctgcactaca gcctgggcga caagagcaag acttcatctc 1355

aaaaaaaaaa aaaaa 1370

<210> 10
<211> 343
<212> PRT
<213> Homo sapiens

<400> 10
Met Trp Ser His Leu Asn Arg Leu Leu Phe Trp Ser Ile Phe Ser Ser
-19 -15 -10 -5

Val Thr Cys Arg Lys Ala Val Leu Asp Cys Glu Ala Met Lys Thr Asn
1 5 10

Glu Phe Pro Ser Pro Cys Leu Asp Ser Lys Thr Lys Val Val Met Lys
15 20 25

Gly Gln Asn Val Ser Met Phe Cys Ser His Lys Asn Lys Ser Leu Gln
30 35 40 45

Ile Thr Tyr Ser Leu Phe Arg Arg Lys Thr His Pro Gly Thr Gln Asp
    50                  55                  60

Gly Lys Gly Glu Pro Ala Ile Phe Asn Leu Ser Ile Thr Glu Ala His
            65                  70                  75

Glu Ser Gly Pro Tyr Lys Cys Lys Ala Gln Val Thr Ser Cys Ser Lys
        80                  85                  90

Tyr Ser Arg Asp Phe Ser Phe Thr Ile Val Asp Pro Val Thr Ser Pro
    95                  100                 105

Val Leu Asn Ile Met Val Ile Gln Thr Glu Thr Asp Arg His Ile Thr
110                 115                 120                 125

Leu His Cys Leu Ser Val Asn Gly Ser Leu Pro Ile Asn Tyr Thr Phe
                130                 135                 140

Phe Glu Asn His Val Ala Ile Ser Pro Ala Ile Ser Lys Tyr Asp Arg
            145                 150                 155

Glu Pro Ala Glu Phe Asn Leu Thr Lys Lys Asn Pro Gly Glu Glu Glu
        160                 165                 170

Glu Tyr Arg Cys Glu Ala Lys Asn Arg Leu Pro Asn Tyr Ala Thr Tyr
    175                 180                 185

Ser His Pro Val Thr Met Pro Ser Thr Gly Gly Asp Ser Cys Pro Phe
190                 195                 200                 205

Cys Leu Lys Leu Leu Leu Pro Gly Leu Leu Leu Leu Leu Val Val Ile
                210                 215                 220

Ile Leu Ile Leu Ala Phe Trp Val Leu Pro Lys Tyr Lys Thr Arg Lys
            225                 230                 235

Ala Met Arg Asn Asn Val Pro Arg Asp Arg Gly Asp Thr Ala Met Glu
        240                 245                 250

Val Gly Ile Tyr Ala Asn Ile Leu Glu Lys Gln Ala Lys Glu Glu Ser
    255                 260                 265

Val Pro Glu Val Gly Ser Arg Pro Cys Val Ser Thr Ala Gln Asp Glu
270                 275                 280                 285

Ala Lys His Ser Gln Glu Leu Gln Tyr Ala Thr Pro Val Phe Gln Glu
                290                 295                 300

Val Ala Pro Arg Glu Gln Glu Ala Cys Asp Ser Tyr Lys Ser Gly Tyr
            305                 310                 315

Val Tyr Ser Glu Leu Asn Phe
        320

<210> 11
<211> 1029
<212> DNA
<213> Homo sapiens

<400> 11
atgtggagcc atttgaacag gctcctcttc tggagcatat tttcttctgt cacttgtaga      60

aaagctgtat tggattgtga ggcaatgaaa acaaatgaat tcccttctcc atgtttggac     120

tcaaagacta aggtggttat gaagggtcaa aatgtatcta tgttttgttc ccataagaac 180

aaatcactgc agatcaccta ttcattgttt cgacgtaaga cacacccggg aacccaggat 240

ggaaaaggtg aacctgcgat ttttaaccta agcatcacag aagcccatga atcaggcccc 300

tacaaatgca aagcccaagt taccagctgt tcaaaataca gtcgtgactt cagcttcacg 360

attgtcgacc cggtgacttc cccagtgctg aacattatgg tcattcaaac agaaacagac 420

cgacatataa cattacattg cctctcagtc aatggctcgc tgcccatcaa ttacactttc 480

tttgaaaacc atgttgccat atcaccagct atttccaagt atgacaggga gcctgctgaa 540

tttaacttaa ccaagaagaa tcctggagaa gaggaagagt ataggtgtga agctaaaaac 600

agattgccta actatgcaac atacagtcac cctgtcacca tgccctcaac aggcggagac 660

agctgtcctt tctgtctgaa gctactactt ccagggttat tactgttgct ggtggtgata 720

atcctaattc tggctttttg ggtactgccc aaatacaaaa caagaaaagc tatgagaaat 780

aatgtgccca gggaccgtgg agacacagcc atggaagttg gaatctatgc aaatatcctt 840

gaaaaacaag caaaggagga atctgtgcca gaagtgggat ccaggccgtg tgtttccaca 900

gcccaagatg aggccaaaca ctcccaggag ctacagtatg ccacccccgt gttccaggag 960

gtggcaccaa gagagcaaga agcctgtgat tcttataaat ctggatatgt ctattctgaa 1020

ctcaacttc 1029

<210> 12
<211> 1370
<212> DNA
<213> Homo sapiens

<220>
<223> Origin; human bone marrow stroma cell HAS 303 - derived clone
OAF038-Pro

<220>
<221> CDS
<222> (7)..(1035)

<220>
<221> sig peptide
<222> (7)..(63)

<220>
<221> mat peptide
<222> (64)..(1035)

<400> 12
gggaga atg tgg agc cat ttg aac agg ctc ctc ttc tgg agc ata ttt 48
Met Trp Ser His Leu Asn Arg Leu Leu Phe Trp Ser Ile Phe
-19 -15 -10

tct tct gtc act tgt aga aaa gct gta ttg gat tgt gag gca atg aaa 96
Ser Ser Val Thr Cys Arg Lys Ala Val Leu Asp Cys Glu Ala Met Lys

-5                 1                   5                   10

aca aat gaa ttc cct tct cca tgt ttg gac tca aag act aag gtg gtt          144
Thr Asn Glu Phe Pro Ser Pro Cys Leu Asp Ser Lys Thr Lys Val Val
                15                  20                  25

atg aag ggt caa aat gta tct atg ttt tgt tcc cat aag aac aaa tca          192
Met Lys Gly Gln Asn Val Ser Met Phe Cys Ser His Lys Asn Lys Ser
            30                  35                  40

ctg cag atc acc tat tca ttg ttt cga cgt aag aca cac ccg gga acc          240
Leu Gln Ile Thr Tyr Ser Leu Phe Arg Arg Lys Thr His Pro Gly Thr
        45                  50                  55

cag gat gga aaa ggt gaa cct gcg att ttt aac cta agc atc aca gaa          288
Gln Asp Gly Lys Gly Glu Pro Ala Ile Phe Asn Leu Ser Ile Thr Glu
 60                  65                  70                  75

gcc cat gaa tca ggc ccc tac aaa tgc aaa gcc caa gtt acc agc tgt          336
Ala His Glu Ser Gly Pro Tyr Lys Cys Lys Ala Gln Val Thr Ser Cys
                 80                  85                  90

tca aaa tac agt cgt gac ttc agc ttc acg att gtc gac ccg gtg act          384
Ser Lys Tyr Ser Arg Asp Phe Ser Phe Thr Ile Val Asp Pro Val Thr
             95                 100                 105

tcc cca gtg ctg aac att atg gtc att caa aca gaa aca gac cga cat          432
Ser Pro Val Leu Asn Ile Met Val Ile Gln Thr Glu Thr Asp Arg His
        110                 115                 120

ata aca tta cat tgc ctc tca gtc aat ggc tcg ctg ccc atc aat tac          480
Ile Thr Leu His Cys Leu Ser Val Asn Gly Ser Leu Pro Ile Asn Tyr
    125                 130                 135

act ttc ttt gaa aac cat gtt gcc ata tca cca gct att tcc aag tat          528
Thr Phe Phe Glu Asn His Val Ala Ile Ser Pro Ala Ile Ser Lys Tyr
140                 145                 150                 155

gac agg gag cct gct gaa ttt aac tta acc aag aag aat cct gga gaa          576
Asp Arg Glu Pro Ala Glu Phe Asn Leu Thr Lys Lys Asn Pro Gly Glu
                160                 165                 170

gag gaa gag tat agg tgt gaa gct aaa aac aga ttg cct aac tat gca          624
Glu Glu Glu Tyr Arg Cys Glu Ala Lys Asn Arg Leu Pro Asn Tyr Ala
            175                 180                 185

aca tac agt cac cct gtc acc atg ccc tca aca ggc gga gac agc tgt          672
Thr Tyr Ser His Pro Val Thr Met Pro Ser Thr Gly Gly Asp Ser Cys
        190                 195                 200

cct ttc tgt ctg aag cta cta ctt cca ggg tta tta ctg ttg ctg gtg          720
Pro Phe Cys Leu Lys Leu Leu Leu Pro Gly Leu Leu Leu Leu Leu Val
    205                 210                 215

gtg ata atc cta att ctg gct ttt tgg gta ctg ccc aaa tac aaa aca          768
Val Ile Ile Leu Ile Leu Ala Phe Trp Val Leu Pro Lys Tyr Lys Thr
220                 225                 230                 235

aga aaa gct atg aga aat aat gtg ccc agg gac cgt gga gac aca gcc          816
Arg Lys Ala Met Arg Asn Asn Val Pro Arg Asp Arg Gly Asp Thr Ala
                240                 245                 250

atg gaa gtt gga atc tat gca aat atc ctt gaa aaa caa gca aag gag          864

Met Glu Val Gly Ile Tyr Ala Asn Ile Leu Glu Lys Gln Ala Lys Glu
            255                 260                 265

gaa tct gtg cca gaa gtg gga tcc agg ccg tgt gtt tcc aca gcc caa      912
Glu Ser Val Pro Glu Val Gly Ser Arg Pro Cys Val Ser Thr Ala Gln
        270                 275                 280

gat gag gcc aaa cac tcc cag gag cta cag tat gcc acc ccc gtg ttc      960
Asp Glu Ala Lys His Ser Gln Glu Leu Gln Tyr Ala Thr Pro Val Phe
    285                 290                 295

cag gag gtg gca cca aga gag caa gaa gcc tgt gat tct tat aaa tct      1008
Gln Glu Val Ala Pro Arg Glu Gln Glu Ala Cys Asp Ser Tyr Lys Ser
300                 305                 310                 315

gga tat gtc tat tct gaa ctc aac ttc tgaaatttac agaaacaaac            1055
Gly Tyr Val Tyr Ser Glu Leu Asn Phe
                320

tacatctcag ggtaaggatg ctttttatga agctgatttc catgaacaaa aagcaaactt    1115

gaggctgagg cgggtggatc acagggtcag gagatcaaga ccatcctggc taacacgatg    1175

aaaccccgtc tctactaaaa aatacaaaaa ttagccaggt gtggtggtgt gtgtgtgtag    1235

tcccagctac tcgggaggct gaggcaggag aatcgcttga gcccgggagg cagaggttgc    1295

agtgagccaa gatcgtgcca ctgcactaca gcctgggcga caagagcaag acttcatctc    1355

aaaaaaaaaa aaaaa                                                     1370

<210> 13
<211> 118
<212> PRT
<213> Homo sapiens

<400> 13
Met Arg Leu Phe Leu Trp Asn Ala Val Leu Thr Leu Phe Val Thr Ser
-20                 -15                 -10                 -5

Leu Ile Gly Ala Leu Ile Pro Glu Pro Glu Val Lys Ile Glu Val Leu
                1               5                   10

Gln Lys Pro Phe Ile Cys His Arg Lys Thr Lys Gly Gly Asp Leu Met
        15                  20                  25

Leu Val His Tyr Glu Gly Tyr Leu Glu Lys Asp Gly Ser Leu Phe His
    30                  35                  40

Ser Thr His Lys His Asn Asn Gly Gln Pro Ile Trp Phe Thr Leu Gly
45                  50                  55                  60

Ile Leu Glu Ala Leu Lys Gly Trp Asp Gln Gly Leu Lys Gly Met Cys
                65                  70                  75

Val Gly Glu Lys Arg Lys Leu Ile Ile Pro Pro Ala Leu Gly Tyr Gly
            80                  85                  90

Lys Glu Gly Lys Val Phe
        95

<210> 14
<211> 354
<212> DNA
<213> Homo sapiens

<400> 14
atgaggcttt tcttgtggaa cgcggtcttg actctgttcg tcacttcttt gattggggct      60

ttgatccctg aaccagaagt gaaaattgaa gttctccaga agccattcat ctgccatcgc     120

aagaccaaag gaggggattt gatgttggtc cactatgaag gctacttaga aaaggacggc     180

tccttatttc actccactca caaacataac aatggtcagc ccatttggtt taccctgggc     240

atcctggagg ctctcaaagg ttgggaccag ggcttgaaag gaatgtgtgt aggagagaag     300

agaaagctca tcattcctcc tgctctgggc tatggaaaag aaggaaaagt cttt           354

<210> 15
<211> 1875
<212> DNA
<213> Homo sapiens

<220>
<223> Origin; human embryonal liver - derived clone OR087H

<220>
<221> CDS
<222> (113)..(466)

<220>
<221> sig peptide
<222> (113)..(172)

<220>
<221> mat peptide
<222> (173)..(466)

<400> 15
   cctgaacttg tctgaagccc ttgtccgtaa gccttgaact acgttcttaa atctatgaag      60
   tcgagggacc tttcgctgct tttgtaggga cttctttcct tgcttcagca ac atg         115
                                                             Met
                                                             -20
   agg ctt ttc ttg tgg aac gcg gtc ttg act ctg ttc gtc act tct ttg       163
   Arg Leu Phe Leu Trp Asn Ala Val Leu Thr Leu Phe Val Thr Ser Leu
                   -15                 -10                 -5
   att ggg gct ttg atc cct gaa cca gaa gtg aaa att gaa gtt ctc cag       211
   Ile Gly Ala Leu Ile Pro Glu Pro Glu Val Lys Ile Glu Val Leu Gln
               1               5                   10
   aag cca ttc atc tgc cat cgc aag acc aaa gga ggg gat ttg atg ttg       259
   Lys Pro Phe Ile Cys His Arg Lys Thr Lys Gly Gly Asp Leu Met Leu
       15                  20                  25
   gtc cac tat gaa ggc tac tta gaa aag gac ggc tcc tta ttt cac tcc       307
   Val His Tyr Glu Gly Tyr Leu Glu Lys Asp Gly Ser Leu Phe His Ser
   30                  35                  40                  45
   act cac aaa cat aac aat ggt cag ccc att tgg ttt acc ctg ggc atc      355
   Thr His Lys His Asn Asn Gly Gln Pro Ile Trp Phe Thr Leu Gly Ile
                   50                  55                  60
   ctg gag gct ctc aaa ggt tgg gac cag ggc ttg aaa gga atg tgt gta      403
   Leu Glu Ala Leu Lys Gly Trp Asp Gln Gly Leu Lys Gly Met Cys Val
               65                  70                  75
   gga gag aag aga aag ctc atc att cct cct gct ctg ggc tat gga aaa      451
   Gly Glu Lys Arg Lys Leu Ile Ile Pro Pro Ala Leu Gly Tyr Gly Lys
           80                  85                  90
   gaa gga aaa gtc ttt tagtacatgc ttgcatgcct cttttggaaa gataccagtt      506
   Glu Gly Lys Val Phe
       95
   ttatcaacaa cctagcgcat gtcacatctc tgtctagatc tgaaatggta aaattccccc      566
   agaaagtaca ctgatattta atattgatct cctggagatt cgaaatggac caagatccca      626
   tgaatcattc caagaaatgg atcttaatga tgactggaaa ctctctaaag atgaggttaa      686
   agcatattta aagaaggagt ttgaaaaaca tggtgcggtg gtgaatgaaa gtcatcatga      746
   tgctttggtg gaggatattt ttgataaaga agatgaagac aaagatgggt ttatatctgc      806
   cagagaattt acatataaac acgatgagtt atagagatac atctaccctt ttaatatagc      866
   actcatcttt caagagaggg cagtcatctt taaagaacat tttattttta tacaatgttc      926
   tttcttgctt tgttttttat ttttatatat tttttctgac tcctatttaa agaacccctt      986
   aggtttctaa gtacccattt ctttctgata agttattggg aagaaaaagc taattggtct     1046
   ttgaatagaa gacttctgga caatttttca ctttcacaga tatgaagctt tgttttactt     1106
   tctcacttat aaatttaaaa tgttgcaact gggaatatac cacgacatga gaccaggtta     1166
   tagcacaaat tagcacccta tatttctgct tccctctatt ttctccaagt tagaggtcaa     1226
   catttgaaaa gccttttgca atagcccaag gcttgctatt ttcatgttat aatgaaatag     1286
   tttatgtgta actggctctg agtctctgct tgaggaccag aggaaaatgg ttgttggacc     1346
   tgacttgtta atggctactg ctttactaag gagatgtgca atgctgaagt tagaaacaag     1406
   gttaatagcc aggcatggtg gctcatgcct gtaatcccag cactttggga ggctgaggcg     1466
   ggcggatcac ctgaggttgg gagttcgaga ccagcctgac caacacggag aaaccctatc     1526
   tctactaaaa atacaaaagt agccgggcgt ggtgatgcgt gcctgtaatc ccagctaccc     1586
   aggaaggctg aggcggcaga atcacttgaa cccggaggcg gaggttgcgg taagccgaga     1646
   tcacctccag cctggacact ctgtctcgaa aaaaagaaaa gaaacacggt taataacata     1706
   taaatatgta tgcattgaga catgctacct aggacttaag ctgatgaagc ttggctccta     1766
   gtgattggtg gcctattatg ataaatagga caaatcattt atgtgtgagt ttctttgtaa     1826
   taaaatgtat caatatgtta aaaaaaaaaa aaaaaaaaaa aaaaaaaaa 1875

<210> 16
<211> 377
<212> PRT
<213> Homo sapiens

<400> 16
Met Asp Ser Ala Leu Ser Asp Pro His Asn Gly Ser Ala Glu Ala Gly
1               5                   10                  15

Gly Pro Thr Asn Ser Thr Thr Arg Pro Pro Ser Thr Pro Glu Gly Ile
            20                  25                  30

Ala Leu Ala Tyr Gly Ser Leu Leu Leu Met Ala Leu Leu Pro Ile Phe
        35                  40                  45

Phe Gly Ala Leu Arg Ser Val Arg Cys Ala Arg Gly Lys Asn Ala Ser
    50                  55                  60

Asp Met Pro Glu Thr Ile Thr Ser Arg Asp Ala Ala Arg Phe Pro Ile
65                  70                  75                  80

Ile Ala Ser Cys Thr Leu Leu Gly Leu Tyr Leu Phe Phe Lys Ile Phe
                85                  90                  95

Ser Gln Glu Tyr Ile Asn Leu Leu Leu Ser Met Tyr Phe Phe Val Leu
            100                 105                 110

Gly Ile Leu Ala Leu Ser His Thr Ile Ser Pro Phe Met Asn Lys Phe
        115                 120                 125

Phe Pro Ala Ser Phe Pro Asn Arg Gln Tyr Gln Leu Leu Phe Thr Gln
    130                 135                 140

Gly Ser Gly Glu Asn Lys Glu Glu Ile Ile Asn Tyr Glu Phe Asp Thr
145                 150                 155                 160

Lys Asp Leu Val Cys Leu Gly Leu Ser Ser Ile Val Gly Val Trp Tyr
                165                 170                 175

Leu Leu Arg Lys His Trp Ile Ala Asn Asn Leu Phe Gly Leu Ala Phe
            180                 185                 190

Ser Leu Asn Gly Val Glu Leu Leu His Leu Asn Asn Val Ser Thr Gly
        195                 200                 205

Cys Ile Leu Leu Gly Gly Leu Phe Ile Tyr Asp Val Phe Trp Val Phe
    210                 215                 220

Gly Thr Asn Val Met Val Thr Val Ala Lys Ser Phe Glu Ala Pro Ile
225                 230                 235                 240

Lys Leu Val Phe Pro Gln Asp Leu Leu Glu Lys Gly Leu Glu Ala Asn
                245                 250                 255

Asn Phe Ala Met Leu Gly Leu Gly Asp Val Val Ile Pro Gly Ile Phe
            260                 265                 270

Ile Ala Leu Leu Leu Arg Phe Asp Ile Ser Leu Lys Lys Asn Thr His
        275                 280                 285

Thr Tyr Phe Tyr Thr Ser Phe Ala Ala Tyr Ile Phe Gly Leu Gly Leu
    290                 295                 300

Thr Ile Phe Ile Met His Ile Phe Lys His Ala Gln Pro Ala Leu Leu
305                 310                 315                 320

Tyr Leu Val Pro Ala Cys Ile Gly Phe Pro Val Leu Val Ala Leu Ala
                325                 330                 335

Lys Gly Glu Val Thr Glu Met Phe Ser Tyr Glu Glu Ser Asn Pro Lys
            340                 345                 350

Asp Pro Ala Ala Val Thr Glu Ser Lys Glu Gly Thr Glu Ala Ser Ala
        355                 360                 365

Ser Lys Gly Leu Glu Lys Lys Glu Lys
    370                 375

<210> 17
<211> 1131
<212> DNA
<213> Homo sapiens

<400> 17
atggactcgg ccctcagcga tccgcataac ggcagtgccg aggcaggcgg ccccaccaac      60

agcactacgc ggccgccttc cacgcccgag ggcatcgcgc tggcctacgg cagcctcctg     120

ctcatggcgc tgctgcccat cttcttcggc gccctgcgct ccgtacgctg cgcccgcggc     180

aagaatgctt cagacatgcc tgaaacaatc accagccggg atgccgcccg cttccccatc     240

atcgccagct gcacactctt ggggctctac ctctttttca aaatattctc ccaggagtac     300

atcaacctcc tgctgtccat gtatttcttc gtgctgggaa tcctggccct gtcccacacc     360

atcagcccct tcatgaataa gttttttcca gccagctttc caaatcgaca gtaccagctg     420

ctcttcacac agggttctgg ggaaaacaag gaagagatca tcaattatga atttgacacc     480

aaggacctgg tgtgcctggg cctgagcagc atcgttggcg tctggtacct gctgaggaag     540

cactggattg ccaacaacct ttttggcctg gccttctccc ttaatggagt agagctcctg     600

cacctcaaca atgtcagcac tggctgcatc ctgctgggcg gactcttcat ctacgatgtc     660

ttctgggtat ttggcaccaa tgtgatggtg acagtggcca agtccttcga ggcaccaata     720

aaattggtgt ttccccagga tctgctggag aaaggcctcg aagcaaacaa ctttgccatg     780

ctgggacttg gagatgtcgt cattccaggg atcttcattg ccttgctgct gcgctttgac     840

atcagcttga agaagaatac ccacacctac ttctacacca gctttgcagc ctacatcttc     900

ggcctgggcc ttaccatctt catcatgcac atcttcaagc atgctcagcc tgccctccta     960

tacctggtcc ccgcctgcat cggttttcct gtcctggtgg cgctggccaa gggagaagtg    1020

acagagatgt tcagttatga ggagtcaaat cctaaggatc cagcggcagt gacagaatcc    1080

aaagagggaa cagaggcatc agcatcgaag gggctggaga agaaagagaa a 1131

<210> 18
<211> 1621
<212> DNA
<213> Homo sapiens

<220>
<223> Origin; human glioblastoma cell line T98G - derived clone OA004FG

<220>
<221> CDS
<222> (117)..(1247)

<400> 18
cacgtcactt cctgttgcct taggggaacg tggctttccc tgcagagccg gtgtctccgc 60

ctgcgtccct gctgcagcaa ccggagctgg agtcggatcc cgaacgcacc ctcgcc 116

atg gac tcg gcc ctc agc gat ccg cat aac ggc agt gcc gag gca ggc 164
Met Asp Ser Ala Leu Ser Asp Pro His Asn Gly Ser Ala Glu Ala Gly
1               5                   10                  15

ggc ccc acc aac agc act acg cgg ccg cct tcc acg ccc gag ggc atc 212
Gly Pro Thr Asn Ser Thr Thr Arg Pro Pro Ser Thr Pro Glu Gly Ile
            20                  25                  30

gcg ctg gcc tac ggc agc ctc ctg ctc atg gcg ctg ctg ccc atc ttc 260
Ala Leu Ala Tyr Gly Ser Leu Leu Leu Met Ala Leu Leu Pro Ile Phe
        35                  40                  45

ttc ggc gcc ctg cgc tcc gta cgc tgc gcc cgc ggc aag aat gct tca 308
Phe Gly Ala Leu Arg Ser Val Arg Cys Ala Arg Gly Lys Asn Ala Ser
    50                  55                  60

gac atg cct gaa aca atc acc agc cgg gat gcc gcc cgc ttc ccc atc 356
Asp Met Pro Glu Thr Ile Thr Ser Arg Asp Ala Ala Arg Phe Pro Ile
65                  70                  75                  80

atc gcc agc tgc aca ctc ttg ggg ctc tac ctc ttt ttc aaa ata ttc 404
Ile Ala Ser Cys Thr Leu Leu Gly Leu Tyr Leu Phe Phe Lys Ile Phe
                85                  90                  95

tcc cag gag tac atc aac ctc ctg ctg tcc atg tat ttc ttc gtg ctg 452
Ser Gln Glu Tyr Ile Asn Leu Leu Leu Ser Met Tyr Phe Phe Val Leu
            100                 105                 110

gga atc ctg gcc ctg tcc cac acc atc agc ccc ttc atg aat aag ttt 500
Gly Ile Leu Ala Leu Ser His Thr Ile Ser Pro Phe Met Asn Lys Phe
        115                 120                 125

ttt cca gcc agc ttt cca aat cga cag tac cag ctg ctc ttc aca cag 548
Phe Pro Ala Ser Phe Pro Asn Arg Gln Tyr Gln Leu Leu Phe Thr Gln
    130                 135                 140

ggt tct ggg gaa aac aag gaa gag atc atc aat tat gaa ttt gac acc 596
Gly Ser Gly Glu Asn Lys Glu Glu Ile Ile Asn Tyr Glu Phe Asp Thr
145                 150                 155                 160

aag gac ctg gtg tgc ctg ggc ctg agc agc atc gtt ggc gtc tgg tac 644
Lys Asp Leu Val Cys Leu Gly Leu Ser Ser Ile Val Gly Val Trp Tyr

                165                 170                 175

ctg ctg agg aag cac tgg att gcc aac aac ctt ttt ggc ctg gcc ttc 692
Leu Leu Arg Lys His Trp Ile Ala Asn Asn Leu Phe Gly Leu Ala Phe
            180                 185                 190

tcc ctt aat gga gta gag ctc ctg cac ctc aac aat gtc agc act ggc 740
Ser Leu Asn Gly Val Glu Leu Leu His Leu Asn Asn Val Ser Thr Gly
        195                 200                 205

tgc atc ctg ctg ggc gga ctc ttc atc tac gat gtc ttc tgg gta ttt 788
Cys Ile Leu Leu Gly Gly Leu Phe Ile Tyr Asp Val Phe Trp Val Phe
    210                 215                 220

ggc acc aat gtg atg gtg aca gtg gcc aag tcc ttc gag gca cca ata 836
Gly Thr Asn Val Met Val Thr Val Ala Lys Ser Phe Glu Ala Pro Ile
225                 230                 235                 240

aaa ttg gtg ttt ccc cag gat ctg ctg gag aaa ggc ctc gaa gca aac 884
Lys Leu Val Phe Pro Gln Asp Leu Leu Glu Lys Gly Leu Glu Ala Asn
                245                 250                 255

aac ttt gcc atg ctg gga ctt gga gat gtc gtc att cca ggg atc ttc 932
Asn Phe Ala Met Leu Gly Leu Gly Asp Val Val Ile Pro Gly Ile Phe
            260                 265                 270

att gcc ttg ctg ctg cgc ttt gac atc agc ttg aag aag aat acc cac 980
Ile Ala Leu Leu Leu Arg Phe Asp Ile Ser Leu Lys Lys Asn Thr His
        275                 280                 285

acc tac ttc tac acc agc ttt gca gcc tac atc ttc ggc ctg ggc ctt 1028
Thr Tyr Phe Tyr Thr Ser Phe Ala Ala Tyr Ile Phe Gly Leu Gly Leu
    290                 295                 300

acc atc ttc atc atg cac atc ttc aag cat gct cag cct gcc ctc cta 1076
Thr Ile Phe Ile Met His Ile Phe Lys His Ala Gln Pro Ala Leu Leu
305                 310                 315                 320

tac ctg gtc ccc gcc tgc atc ggt ttt cct gtc ctg gtg gcg ctg gcc 1124
Tyr Leu Val Pro Ala Cys Ile Gly Phe Pro Val Leu Val Ala Leu Ala
                325                 330                 335

aag gga gaa gtg aca gag atg ttc agt tat gag gag tca aat cct aag 1172
Lys Gly Glu Val Thr Glu Met Phe Ser Tyr Glu Glu Ser Asn Pro Lys
            340                 345                 350

gat cca gcg gca gtg aca gaa tcc aaa gag gga aca gag gca tca gca 1220
Asp Pro Ala Ala Val Thr Glu Ser Lys Glu Gly Thr Glu Ala Ser Ala
        355                 360                 365

tcg aag ggg ctg gag aag aaa gag aaa tgatgcggct ggtgcccgag 1267
Ser Lys Gly Leu Glu Lys Lys Glu Lys
    370                 375

cctctcaggg ccagaccaga cagatggggg ctgggcccac acaggcgtgc accggtagag 1327

ggcacaggag gccaagggca gctccaggac agggcagggg gcagcaggat acctccagcc 1387

aggcctctgt ggcctctgtt tccttctccc tttcttggcc ctcctctgct cctccccaca 1447

ccctgcaggc aaaagaaacc cccagcttcc cccctccccg ggagccaggt gggaaaagtg 1507

ggtgtgattt ttagattttg tattgtggac tgattttgcc tcacattaaa aactcatccc 1567

atggccaggg cgggccactg tgctcctgaa aaaaaaaaaa aaaaa 1612

<210> 19
<211> 377
<212> PRT
<213> Homo sapiens

<400> 19
Met Asp Ser Ala Leu Ser Asp Pro His Asn Gly Ser Ala Glu Ala Gly
1               5                   10                  15

Gly Pro Thr Asn Ser Thr Thr Arg Pro Pro Ser Thr Pro Glu Gly Ile
            20                  25                  30

Ala Leu Ala Tyr Gly Ser Leu Leu Leu Met Ala Leu Leu Pro Ile Phe
        35                  40                  45

Phe Gly Ala Leu Arg Ser Val Arg Cys Ala Arg Gly Lys Asn Ala Ser
    50                  55                  60

Asp Met Pro Glu Thr Ile Thr Ser Arg Asp Ala Ala Arg Phe Pro Ile
65                  70                  75                  80

Ile Ala Ser Cys Thr Leu Leu Gly Leu Tyr Leu Phe Phe Lys Ile Phe
                85                  90                  95

Ser Gln Glu Tyr Ile Asn Leu Leu Leu Ser Met Tyr Phe Phe Val Leu
            100                 105                 110

Gly Ile Leu Ala Leu Ser His Thr Ile Ser Pro Phe Met Asn Lys Phe
        115                 120                 125

Phe Pro Ala Ser Leu Pro Asn Arg Gln Tyr Gln Leu Leu Phe Thr Gln
    130                 135                 140

Gly Ser Gly Glu Asn Lys Glu Glu Ile Ile Asn Tyr Glu Phe Asp Thr
145                 150                 155                 160

Lys Asp Leu Val Cys Leu Gly Leu Ser Ser Ile Val Asp Val Trp Tyr
                165                 170                 175

Leu Leu Arg Lys His Trp Ile Ala Asn Asn Leu Phe Gly Leu Ala Phe
            180                 185                 190

Ser Leu Asn Gly Val Glu Leu Leu His Leu Asn Asn Val Ser Thr Gly
        195                 200                 205

Cys Ile Leu Leu Gly Gly Leu Phe Ile Tyr Asp Val Phe Trp Val Phe
    210                 215                 220

Gly Thr Asn Val Met Val Thr Val Ala Lys Ser Phe Glu Ala Pro Ile
225                 230                 235                 240

Lys Leu Val Phe Pro Gln Asp Leu Leu Glu Lys Gly Leu Glu Ala Asn
                245                 250                 255

Asn Phe Ala Met Leu Gly Leu Gly Asp Val Val Ile Pro Gly Ile Phe
            260                 265                 270

Ile Ala Leu Leu Leu Arg Phe Asp Ile Ser Leu Lys Lys Asn Thr His

275                 280                 285

Thr Tyr Phe Tyr Thr Ser Phe Ala Ala Tyr Ile Phe Gly Leu Gly Leu
    290                 295                 300

Thr Ile Phe Ile Met His Ile Phe Lys His Ala Gln Pro Ala Leu Leu
305                 310                 315                 320

Tyr Leu Val Pro Ala Cys Ile Gly Phe Pro Val Leu Val Ala Leu Ala
                325                 330                 335

Lys Gly Glu Val Thr Glu Met Phe Ser Tyr Glu Glu Ser Asn Pro Lys
            340                 345                 350

Asp Pro Ala Ala Val Thr Glu Ser Lys Glu Gly Thr Glu Ala Ser Ala
        355                 360                 365

Ser Lys Gly Leu Glu Lys Lys Glu Lys
    370                 375

<210> 20
<211> 1131
<212> DNA
<213> Homo sapiens

<400> 20
atggactcgg ccctcagcga tccgcataac ggcagtgccg aggcaggcgg ccccaccaac      60

agcactacgc ggccgccttc cacgcccgag ggcatcgcgc tggcctacgg cagcctcctg     120

ctcatggcgc tgctgcccat cttcttcggc gccctgcgct ccgtacgctg cgcccgcggc     180

aagaatgctt cagacatgcc tgaaacaatc accagccggg atgccgcccg cttccccatc     240

atcgccagct gcacactctt ggggctctac ctctttttca aaatattctc ccaggagtac     300

atcaacctcc tgctgtccat gtatttcttc gtgctgggaa tcctggccct gtcccacacc     360

atcagcccct tcatgaataa gttttttcca gccagccttc caaatcgaca gtaccagctg     420

ctcttcacac agggttctgg ggaaaacaag gaagagatca tcaattatga atttgacacc     480

aaggacctgg tgtgcctggg cctgagcagc atcgttgacg tctggtacct gctgaggaag     540

cactggattg ccaacaacct ttttggcctg gccttctccc ttaatggagt agagctcctg     600

cacctcaaca atgtcagcac tggctgcatc ctgctgggcg gactcttcat ctacgatgtc     660

ttctgggtat ttggcaccaa tgtgatggtg acagtggcca agtccttcga ggcaccaata     720

aaattggtgt ttccccagga tctgctggag aaaggcctcg aagcaaacaa ctttgccatg     780

ctgggacttg gagatgtcgt cattccaggg atcttcattg ccttgctgct gcgctttgac     840

atcagcttga agaagaatac ccacacctac ttctacacca gctttgcagc ctacatcttc     900

ggcctgggcc ttaccatctt catcatgcac atcttcaagc atgctcagcc tgccctccta     960

tacctggtcc ccgcctgcat cggttttcct gtcctggtgg cgctggccaa gggagaagtg    1020

acagagatgt tcagttatga ggagtcaaat cctaaggatc cagcggcagt gacagaatcc    1080

aaagagggaa cagaggcatc agcatcgaag gggctggaga agaaagagaa a          1131

<210> 21
<211> 1621
<212> DNA
<213> Homo sapiens

<220>
<223> Origin; human glioblastoma cell line T98G - derived clone OA004LD

<220>
<221> CDS
<222> (117)..(1247)

<400> 21
cacgtcactt cctgttgcct taggggaacg tggctttccc tgcagagccg gtgtctccgc      60

ctgcgtccct gctgcagcaa ccggagctgg agtcggatcc cgaacgcacc ctcgcc        116

atg gac tcg gcc ctc agc gat ccg cat aac ggc agt gcc gag gca ggc      164
Met Asp Ser Ala Leu Ser Asp Pro His Asn Gly Ser Ala Glu Ala Gly
1               5                   10                  15

ggc ccc acc aac agc act acg cgg ccg cct tcc acg ccc gag ggc atc      212
Gly Pro Thr Asn Ser Thr Thr Arg Pro Pro Ser Thr Pro Glu Gly Ile
            20                  25                  30

gcg ctg gcc tac ggc agc ctc ctg ctc atg gcg ctg ctg ccc atc ttc      260
Ala Leu Ala Tyr Gly Ser Leu Leu Leu Met Ala Leu Leu Pro Ile Phe
        35                  40                  45

ttc ggc gcc ctg cgc tcc gta cgc tgc gcc cgc ggc aag aat gct tca      308
Phe Gly Ala Leu Arg Ser Val Arg Cys Ala Arg Gly Lys Asn Ala Ser
    50                  55                  60

gac atg cct gaa aca atc acc agc cgg gat gcc gcc cgc ttc ccc atc      356
Asp Met Pro Glu Thr Ile Thr Ser Arg Asp Ala Ala Arg Phe Pro Ile
65                  70                  75                  80

atc gcc agc tgc aca ctc ttg ggg ctc tac ctc ttt ttc aaa ata ttc      404
Ile Ala Ser Cys Thr Leu Leu Gly Leu Tyr Leu Phe Phe Lys Ile Phe
                85                  90                  95

tcc cag gag tac atc aac ctc ctg ctg tcc atg tat ttc ttc gtg ctg      452
Ser Gln Glu Tyr Ile Asn Leu Leu Leu Ser Met Tyr Phe Phe Val Leu
            100                 105                 110

gga atc ctg gcc ctg tcc cac acc atc agc ccc ttc atg aat aag ttt      500
Gly Ile Leu Ala Leu Ser His Thr Ile Ser Pro Phe Met Asn Lys Phe
        115                 120                 125

ttt cca gcc agc ctt cca aat cga cag tac cag ctg ctc ttc aca cag      548
Phe Pro Ala Ser Leu Pro Asn Arg Gln Tyr Gln Leu Leu Phe Thr Gln
    130                 135                 140

ggt tct ggg gaa aac aag gaa gag atc atc aat tat gaa ttt gac acc      596
Gly Ser Gly Glu Asn Lys Glu Glu Ile Ile Asn Tyr Glu Phe Asp Thr
145                 150                 155                 160

aag gac ctg gtg tgc ctg ggc ctg agc agc atc gtt gac gtc tgg tac      644

Lys Asp Leu Val Cys Leu Gly Leu Ser Ser Ile Val Asp Val Trp Tyr
                165                 170                 175

ctg ctg agg aag cac tgg att gcc aac aac ctt ttt ggc ctg gcc ttc 692
Leu Leu Arg Lys His Trp Ile Ala Asn Asn Leu Phe Gly Leu Ala Phe
            180                 185                 190

tcc ctt aat gga gta gag ctc ctg cac ctc aac aat gtc agc act ggc 740
Ser Leu Asn Gly Val Glu Leu Leu His Leu Asn Asn Val Ser Thr Gly
        195                 200                 205

tgc atc ctg ctg ggc gga ctc ttc atc tac gat gtc ttc tgg gta ttt 788
Cys Ile Leu Leu Gly Gly Leu Phe Ile Tyr Asp Val Phe Trp Val Phe
    210                 215                 220

ggc acc aat gtg atg gtg aca gtg gcc aag tcc ttc gag gca cca ata 836
Gly Thr Asn Val Met Val Thr Val Ala Lys Ser Phe Glu Ala Pro Ile
225                 230                 235                 240

aaa ttg gtg ttt ccc cag gat ctg ctg gag aaa ggc ctc gaa gca aac 884
Lys Leu Val Phe Pro Gln Asp Leu Leu Glu Lys Gly Leu Glu Ala Asn
                245                 250                 255

aac ttt gcc atg ctg gga ctt gga gat gtc gtc att cca ggg atc ttc 932
Asn Phe Ala Met Leu Gly Leu Gly Asp Val Val Ile Pro Gly Ile Phe
            260                 265                 270

att gcc ttg ctg ctg cgc ttt gac atc agc ttg aag aag aat acc cac 980
Ile Ala Leu Leu Leu Arg Phe Asp Ile Ser Leu Lys Lys Asn Thr His
        275                 280                 285

acc tac ttc tac acc agc ttt gca gcc tac atc ttc ggc ctg ggc ctt 1028
Thr Tyr Phe Tyr Thr Ser Phe Ala Ala Tyr Ile Phe Gly Leu Gly Leu
    290                 295                 300

acc atc ttc atc atg cac atc ttc aag cat gct cag cct gcc ctc cta 1076
Thr Ile Phe Ile Met His Ile Phe Lys His Ala Gln Pro Ala Leu Leu
305                 310                 315                 320

tac ctg gtc ccc gcc tgc atc ggt ttt cct gtc ctg gtg gcg ctg gcc 1124
Tyr Leu Val Pro Ala Cys Ile Gly Phe Pro Val Leu Val Ala Leu Ala
                325                 330                 335

aag gga gaa gtg aca gag atg ttc agt tat gag gag tca aat cct aag 1172
Lys Gly Glu Val Thr Glu Met Phe Ser Tyr Glu Glu Ser Asn Pro Lys
            340                 345                 350

gat cca gcg gca gtg aca gaa tcc aaa gag gga aca gag gca tca gca 1220
Asp Pro Ala Ala Val Thr Glu Ser Lys Glu Gly Thr Glu Ala Ser Ala
        355                 360                 365

tcg aag ggg ctg gag aag aaa gag aaa tgatgcggct ggtgcccgag 1267
Ser Lys Gly Leu Glu Lys Lys Glu Lys
    370                 375

cctctcaggg ccagaccaga cagatggggg ctgggcccac acaggcgtgc accggtagag 1327

ggcacaggag gccaagggca gctccaggac agggcagggg gcagcaggat acctccagcc 1387

aggcctctgt ggcctctgtt tccttctccc tttcttggcc ctcctctgct cctccccaca 1447

ccctgcaggc aaaagaaacc cccagcttcc cccctccccg ggagccaggt gggaaaagtg 1587

ggtgtgattt ttagattttg tattgtggac tgattttgcc tcacattaaa aactcatccc 1567

atggccaggg cgggccactg tgctcctgaa aaaaaaaaaa aaaaa 1612

<210> 22
<211> 35
<212> DNA
<213> Artificial Sequence

<220>
<223> Description of Artificial Sequence:Primer

<400> 22
cgattgaatt ctagacctgc ctcgagnnnn nnnnn 35

<210> 23
<211> 27
<212> DNA
<213> Artificial Sequence

<220>
<223> Description of Artificial Sequence:Primer OM007-F3

<400> 23
aactgcagat cttgggactc atcagcc 27

<210> 24
<211> 28
<212> DNA
<213> Artificial Sequence

<220>
<223> Description of Artificial Sequence:Primer OM007-F2

<400> 24
aagaggacat tgttttcatc atggatgc 28

<210> 25
<211> 27
<212> DNA
<213> Artificial Sequence

<220>
<223> Description of Artificial Sequence:Primer OMB096-F1

<400> 25
acaacatgca ccaccagtgg cttctgc 27

<210> 26
<211> 28
<212> DNA
<213> Artificial Sequence

<220>
<223> Description of Artificial Sequence:Primer OAF038-F1

<400> 26

agaatgtgga gccatttgaa caggctcc                                          28

<210> 27
<211> 27
<212> DNA
<213> Artificial Sequence

<220>
<223> Description of Artificial Sequence:Primer OR087H-F1

<400> 27
tgaagccctt gtccgtaagc cttgaac                                          27

<210> 28
<211> 24
<212> DNA
<213> Artificial Sequence

<220>
<223> Description of Artificial Sequence:Primer OA004-F1

<220>
<221> modified base
<222> 1
<223> biotin conjugated base

<400> 28
atgcacatct tcaagcatgc tcag                                             24